# EUROPÄISCHE PATENTSCHRIFT

(11) **EP 3 609 995 B1**
(45) Veröffentlichungstag und Bekanntmachung des Hinweises auf die Patenterteilung: **25.11.2020**
(21) Anmeldenummer: 18716316.7
(22) Anmeldetag: 13.04.2018
(51) Int. Cl.: C12M 1/32, C12M 1/00, C12M 1/26, C12M 1/34, B01L 3/00

(54) **VERFAHREN ZUM PROZESSIEREN EINER FLÜSSIGEN PROBE**
METHOD FOR PROCESSING A LIQUID SAMPLE
PROCÉDÉ DE TRAITEMENT D'UN ÉCHANTILLON LIQUIDE

(30) Priorität: 13.04.2017 LU 100170
(43) Veröffentlichungstag der Anmeldung: 19.02.2020
(73) Patentinhaber: cytena Bioprocess Solutions co., Ltd., Taipei City 110 (TW)
(72) Erfinder: SCHÖNDUBE, Jonas, 79108 Freiburg (DE); CHENGHAN, Tsai, 79098 Freiburg (DE); GROSS, Andre, 79104 Freiburg (DE); ZIMMERMANN, Stefan, 79283 Bollschweil (DE); KOLTAY, Peter, 79117 Freiburg (DE)
(74) Vertreter: Dennemeyer & Associates S.A.
(86) Internationale Anmeldenummer: PCT/EP2018/059601
(87) Internationale Veröffentlichungsnummer: WO 2018/189397

(56) Entgegenhaltungen:
- EP-A1- 2 927 312
- WO-A1-02/072423
- WO-A1-2011/140071
- WO-A1-2013/019212
- DE-A1- 19 742 163
- DE-A1-102006 030 068
- US-A- 2 956 931
- US-A- 5 839 828
- US-A1- 2012 149 603

## Beschreibung

Die Erfindung betrifft ein Verfahren zum Prozessieren einer in einem Behältnis befindlichen flüssigen Probe, wobei an dem Behältnis eine Aufsatzvorrichtung derart befestigt wird, dass wenigstens eine Fluidleitung in die flüssige Probe ragt und durch die Fluidleitung ein Fluid unmittelbar in die flüssige Probe dispensiert wird und/oder ein Teil der flüssigen Prob) in die Fluidleitung eingesaugt wird.

Darüber hinaus betrifft die Erfindung eine Aufsatzvorrichtung, die an einem Behältnis zur Aufnahme einer flüssigen Probe wieder lösbar befestigbar ist, mit wenigstens einer Fluidleitung, die derart ausgebildet und dazu bestimmt ist, dass die Fluidleitung in die flüssige Probe ragt und dass durch die Fluidleitung unmittelbar ein Fluid in die flüssige Probe dispensierbar ist und/oder ein Teil der flüssigen Probe in die Fluidleitung einsaugbar ist. Außerdem betrifft die Erfindung eine Vorrichtung mit der erfindungsgemäßen Aufsatzvorrichtung und einem Behältnis zum Aufnehmen der flüssigen Probe.

Aus dem Stand der Technik ist bekannt, dass Wirkstoffe, wie beispielsweise monoklonale Antikörper und andere Proteine mit Hilfe sogenannter monoklonaler Zelllinien hergestellt werden. Dies sind Populationen aus Zellen, die alle von einer einzelnen Mutterzelle abstammen. Das Herstellen von monoklonalen Zelllinien ist notwendig, da nur so sichergestellt werden kann, dass alle Zellen der Population ein annährend gleiches Genom haben, um die Wirkstoffe zu erzeugen.

Um eine monoklonale Zelllinie zu erzeugen, werden Zellen einzeln in Behältnisse einer Mikrotiterplatte überführt. Die überführten Zellen werden hergestellt, indem eine Host-Zelllinie genetisch verändert wird und diese veränderten Zellen vereinzelt werden. Das Ablegen einzelner Zellen in die Mikrotiterplatten geschieht durch beispielsweise Freistrahldruckmethoden oder Pipettieren.

Danach werden Zellkolonien, die aus einer Zelle wachsen, in den Behältnissen der Mikrotiterplatte statisch, das heißt ohne Bewegung, kultiviert, bis sie fast den gesamten Boden der Behältnisse der Mikrotiterplatte bedecken. Anschließend werden die Zellkulturen schrittweise in größere Gefäße überführt. Insbesondere werden die Zellkulturen in unterschiedlich große Mikrotiterplatten und im Anschluss daran in einen Schüttelkolben und letztendlich in den Bioreaktor überführt. Typischerweise wird beim Schüttelkolben von statischer Kultur zu dynamischer Kultur gewechselt, dass heißt, dass die Schüttelkolben kontinuierlich geschüttelt werden, um die Zellkultur zu mischen. Schlussendlich wird aus einer Serie von vielen hundert bis tausend solcher Zellkulturen diejenige in die Produktion überführt, die die Wirkstoffe am stabilsten und in größter Menge in einem Bioreaktor herstellen kann.

Im Bioreaktor wird typischerweise die Zellkultur in Bewegung gehalten, der pH-Wert, der Sauerstoff- und Nährwertgehalt und die Temperatur eingestellt, um optimale Wachstumsbedingungen für die Zellen zu schaffen. Zudem lassen sich in einem bewegten Nährmedium mit schwimmenden Zellen mehr Zellen pro Volumen züchten. Dies erhöht die Produktionsmenge bei gleichbleibenden Volumen gegenüber ruhenden Zellkulturen deutlich.

Die statische Kultivierung in Mikrotiterplatten ist nicht ideal für die Zellen, da diese so gezüchtet sind, dass sie sich in geschüttelter oder gerüttelter Umgebung ideal verhalten. Bei einem Überführen der Zellen in statische Bedingungen kann es zu ungewollten Kulturverhalten kommen, wie beispielsweise der Reduktion der metabolischen Aktivität und schlimmstenfalls dem Absterben der Zellen kommen. Man kann Zellen jedoch nicht von Anfang an in Bioreaktoren züchten, da die Zellkulturen bei geringen Konzentrationen nicht wachsen. So vermehren sich Einzelzellen nicht in großen Volumina. In der Regel hat dies das Absterben der Zelle zur Folge. Daher ist eine schrittweise Erhöhung des Volumens, in dem sich die Zelle befindet, notwendig.

Die Menge an vitalen, sich vermehrenden Kolonien und dem daraus gewonnenen Produkt sind essentiell für die Industrie. Sie bestimmen den Umsatz, den man mit einer Produktionscharge an Zellen generieren kann.

Aus dem Stand der Technik sind Vorrichtungen bekannt, die Schüttler aufweisen, die die Mikrotiterplatten schütteln und somit die statischen Bedingungen in der Mikrotiterplatte verhindert werden. Nachteilig an den bekannten Ausführungen ist jedoch, dass ein Schütteln der Mikrotiterplatte bei Behältnissen praktisch nicht mehr möglich ist, die ein kleines Volumen aufweisen.

WO 02/072423 A1 offenbart einen Mikroplattendeckel, der eine kontrollierte Abgabe kleiner Mengen von Proben und Reagenzien an eine Mikroplatte ermöglicht und die Verdunstung während der Verarbeitung verhindert.

EP 2 927 312 A1 offenbart ein automatisiertes zellfreies Proteinproduktionssystem und ein Verfahren zur Herstellung eines Proteins unter Verwendung des Proteinproduktionssystems.

DE 10 2006 030 068 A1 offenbart ein Verfahren zur Zu- und Abfuhr von Flüssigkeiten in Mikroreaktorenarrays durch einen oder mehrere Fluidkanäle, die in jeden einzelnen Mikroreaktor führen und individuell gesteuert und geregelt werden können. Die zu- oder abgeführten Flüssigkeitsmengen werden während eines kontinuierlichen Schüttelvorgangs in das Volumen der Reaktionsflüssigkeit ein- oder abgeleitet und durch das Schütteln jeweils gleichmäßig gemischt.

US 2012/149 603 A1 offenbart ein integriertes Probenanalysesystem. Dieses enthält ein Probenvorbereitungs-/analysemodul mit einer Probenreinigungsvorrichtung und einer Probenanalysevorrichtung, die ein Mikroarray umfasst, ein Temperaturkontrollmodul und eine Abbildungsvorrichtung.

US 5,839,828 A offenbart einen statischen Mischer. Der Mischer ist zur Anordnung in einem Rohr mit einer Fluidströmungsrichtung geeignet und weist einen Umfangsflansch auf, der sich von der inneren Rohroberfläche radial nach innen erstreckt und seinerseits mindestens ein Paar gegenüberliegender Klappen aufweist, die sich von dort aus erstrecken und in Richtung der Fluidströmung geneigt sind.

Die Aufgabe der Erfindung besteht daher darin, ein Verfahren anzugeben, mittels dem, unabhängig von dem Volumen des Behältnisses, die zuvor genannten Nachteile vermieden werden können.

Die Aufgabe der Erfindung wird gelöst durch ein Verfahren nach dem Oberbegriff des Anspruchs 1, das dadurch gekennzeichnet ist, dass das dispensierte Fluid ein zuvor eingesaugter Teil der flüssigen Probe ist und/oder dass das dispensierte Fluid ein zuvor aus der flüssigen Probe eingesaugtes Gas ist.

Darüber hinaus besteht eine Aufgabe der Erfindung darin, eine Vorrichtung anzugeben, bei der, unabhängig von dem Volumen des Behältnisses, die zuvor genannten Nachteile vermieden werden können.

Die Aufgabe ist gelöst durch eine Aufsatzvorrichtung nach dem Oberbegriff des Anspruchs 7, die dadurch gekennzeichnet ist, dass die Aufsatzvorrichtung eine Steuervorrichtung aufweist, die ausgelegt ist, zu bewirken, dass das dispensierte Fluid ein zuvor eingesaugter Teil der flüssigen Probe ist und/oder dass das dispensierte Fluid ein zuvor aus der flüssigen Probe eingesaugtes Gas ist.

Das erfindungsgemäße Verfahren und die Vorrichtung weisen den Vorteil auf, dass schon sehr früh in dem Produktionsprozess die optimalen Produktionsbedingungen für das Zellwachstum realisiert werden. Insbesondere lässt sich bei dem erfindungsgemäßen Verfahren und der erfindungsgemäßen Vorrichtung eine Bewegung und/oder ein Durchmischen der flüssigen Probe realisieren und/oder der Gasgehalt in der flüssigen Probe und/oder der Nährstoffgehalt der flüssigen Probe und/oder die Zellkonzentration der flüssigen Probe kann eingestellt werden.

Dies ist möglich, weil die Fluidleitung in die flüssige Probe eindringt und mittels der Fluidleitung ein Einsaugen eines Teils der flüssigen Probe oder ein unmittelbares Dispensieren eines Fluids in die flüssige Probe realisiert werden kann.

Die flüssige Probe kann eine flüssige biologische oder chemische Probe sein. Insbesondere kann die flüssige Probe Zellen aufweisen, die in einer Flüssigkeit schwimmen. Das nicht zur Aufsatzvorrichtung gehörende Behältnis kann ein Mikrobioreaktor sein. In einem Mikrobioreaktor können zum Prozessieren der Probe bestimmte chemische und/oder biologische Reaktionen unter definierten Bedingungen ablaufen, wobei die Reaktionen unter anderem durch Zugabe und/oder Abfuhr von Fluiden steuerbar oder regelbar sind. Insbesondere können in dem Mikrobioreaktor beispielsweise Zellen kultiviert werden.

Die Fluidleitung kann rigid ausgeführt sein. Insbesondere kann die Fluidleitung eine Kanüle sein. Das Fluid kann ein Gas oder eine Flüssigkeit, insbesondere die flüssige Probe, sein und ist beweglich und kann daher durch Pumpen, Ventile, Fluidleitungen, etc., geleitet und transportiert werden. Mittels der Aufsatzvorrichtung kann somit Gas oder Flüssigkeit dispensiert werden. Eine fluidische Verbindung zwischen zwei Bauteilen besteht, wenn das Fluid von einem Bauteil in das andere Bauteile strömen kann. Als Durchmischen der flüssigen Probe wird ein Vorgang verstanden, bei denen die Bestandteile der flüssigen Probe derart relativ zueinander bewegt werden, dass ein neues Anordnungsschema entsteht.

Ganz besonders vorteilhaft ist, wenn das Einsaugen und Dispensieren mehrmals hintereinander ausgeführt wird, um die flüssige Probe zu durchmischen und/oder das Einsaugen und Dispensieren abwechselnd ausgeführt wird, um die flüssige Probe zu durchmischen. Die Aufsatzvorrichtung kann eine Steuervorrichtung aufweisen oder mit der Steuervorrichtung verbunden sein. Die Steuervorrichtung kann ausgelegt sein, um ein Einsaugen und Dispensieren mehrmals hintereinander und abwechselnd zu bewirken, um die flüssige Probe zu durchmischen. Insbesondere kann die Steuervorrichtung durch entsprechendes Steuern einer Pumpe bewirken, dass das Einsaugen und Dispensieren mehrmals hintereinander und/oder abwechselnd ausgeführt wird. Ein abwechselndes Einsaugen und Dispensieren kann durch ein reziprokes Pumpen realisiert werden. Insbesondere kann ein Pumpenelement zum Durchmischen der flüssigen Probe reziprok bewegt werden.

Durch das Durchmischen der flüssigen Probe ist sichergestellt, dass keine statischen Bedingungen in dem Behältnis herrschen. Dies bedeutet, dass von Anfang an ideale Produktionsbedingungen realisiert werden können, so dass ein schnelles Wachstum von beispielsweise Zellen realisiert werden kann. Darüber hinaus ist die Produktivität besser vorhersehbar. Außerdem werden eine bessere Stabilität der Zellkultur und eine höhere Zelldichte als bei Ausführungen erreicht, bei denen die Zellkulturen unter statischen Bedingungen kultiviert werden.

Dabei kann die Menge der eingesaugten flüssigen Probe zwischen 5% und 30% der Gesamtmenge der flüssigen Probe betragen. Außerdem kann der Vorgang des Einsaugens und Absaugens wenigstens 3 mal, insbesondere kontinuierlich für eine vorgegebene Zeitdauer, wiederholt werden. Dadurch lassen sich ganz besonders vorteilhafte Bedingungen für das Zellwachstum realisieren.

Bei einer besonderen Ausführung kann das in die flüssige Probe dispensierte Fluid ein Gas sein. Dabei kann das dispensierte Gas ein zuvor aus der flüssigen Probe eingesaugtes Gas sein. Bei dem eingesaugten Gas kann es sich um das Gas handeln, das zeitlich vorher, insbesondere aus einem Gastank oder der Umgebung, unter Verwendung der Pumpe und mittels der Fluidleitung in die flüssige Probe dispensiert wurde. Alternativ kann es sich bei dem eingesaugten Gas um Gas aus einer nachstehend beschriebenen Gasblase handeln.

Insbesondere kann das Fluid Sauerstoff oder Kohlenstoffdioxid sein. Sauerstoff ist wichtig für das Zellwachstum und Kohlenstoffdioxid kann eingesetzt werden, um den pH-Wert einzustellen. Bei einer Gaszufuhr in die flüssige Probe können die Gasblasen in der flüssigen Probe aufsteigen. Die Gaszufuhr kann nach Abschluss des Durchmischens der flüssigen Probe infolge des Vorgangs des Einsaugens und Dispensierens der flüssigen Probe erfolgen.

Ganz besonders vorteilhaft ist eine Ausführung, bei der die Gasblase erzeugt wird, wobei ein Gasblasendurchmesser vergrößert und verkleinert wird, um die flüssige Probe zu durchmischen. Insbesondere kann die Gasblase an einem Auslas der Fluidleitung erzeugt werden. Ein Vergrößern des Gasblasendurchmessers kann durch Dispensieren des zuvor eingesaugten Gases aus der Fluidleitung realisiert werden. Ein Verkleinern des Gasdurchmessers kann durch Einsaugen eines Teils des Gases aus der Gasblase oder des gesamten Gases aus der Gasblase in die Fluidleitung realisiert werden. Das Vergrößern und Verkleinern des Gasblasendurchmessers kann mehrmals hintereinander und/oder abwechselnd erfolgen. Dadurch kann die Durchmischung der flüssigen Probe verbessert werden. Insbesondere kann die Menge des eingesaugten Gases zwischen 50% und 100% der Gesamtmenge der Gasblase betragen und/oder der Vorgang des Einsaugens und Dispensierens kann wenigstens 3 mal, insbesondere kontinuierlich für eine vorgegebene Zeitdauer, wiederholt werden.

Im Ergebnis kann mittels der Aufsatzvorrichtung ein Durchmischen der flüssigen Probe einerseits durch abwechselndes Dispensieren der flüssigen Probe aus der Fluidleitung und Einsaugen eines Teils der flüssigen Probe in die Fluidleitung und andererseits durch Vergrößern und Verkleinern des Gasblasendurchmessers realisiert werden. Beide Vorgänge können zeitgleich bei einer Aufsatzvorrichtung, die mehrere Fluidleitungen aufweist, durchgeführt werden. Alternativ können die Vorgänge zeitversetzt durchgeführt werden.

Der Gasgehalt in der flüssigen Probe kann durch, insbesondere kontrollierte, Zufuhr des Gases in die flüssige Probe eingestellt werden. Dadurch lässt sich ein Wachstum der Zellkultur erhöhen. Das Gas kann in einem Gastank der Vorrichtung gelagert werden, der mit der Fluidleitung fluidisch verbunden ist. Darüber hinaus kann die Vorrichtung eine Einstellvorrichtung, wie beispielsweise ein Gastankventil, aufweisen, mittels der das in die Fluidleitung zugeführte Gas eingestellt werden kann.

Dabei kann bei einer, insbesondere kontrollierten, Zufuhr von Gas in die flüssige Probe der Gasgehalt der flüssigen Probe durch diffusiven Austausch zwischen dem in die flüssige Probe dispensierten Gas und der flüssige Probe eingestellt werden. Diese Ausführung ist besonders vorteilhaft, wenn das Behältnis ein kleines Volumen aufweist. Dabei kann das Gas derart in die flüssige Probe zugeführt werden, dass dieses in der flüssigen Probe aufsteigt. Alternativ kann am Auslass der Fluidleitung die Gasblase erzeugt werden. Die Gasblase kann einen großen Durchmesser und damit eine große Kontaktfläche aufweisen. Aufgrund der großen Kontaktfläche kann der diffusive Austausch zwischen dem Gas und der flüssigen Probe ganz besonders gut erfolgen.

Alternativ oder zusätzlich kann der Gasgehalt durch diffusiven Austausch zwischen dem in der Fluidleitung befindlichen Gas und der flüssigen Probe eingestellt werden. Bei dieser Ausführung wird ein Gasaufstieg durch eine besondere Ausbildung der Fluidleitung verhindert. Dazu können sich von einer Wand der Fluidleitung mehrere Finger, insbesondere genau drei, in Längsrichtung der Fluidleitung erstrecken. Die einzelnen Finger können in Umfangsrichtung der Fluidleitung beabstandet zueinander angeordnet sein.

Alternativ oder zusätzlich kann der Gasgehalt der flüssigen Probe durch diffusiven Austausch zwischen dem in einem Abschnitt der Fluidleitung befindlichen Gas und der in die Fluidleitung eingesaugten flüssigen Probe eingestellt werden. Bei dieser Ausführung ist eine Ausbildung der Fluidleitung vorteilhaft, bei der eine Wand der Fluidleitung mehrere, insbesondere ringförmige, Vorsprünge aufweist, die in Längsrichtung der Fluidleitung voneinander beabstandet angeordnet sind. Die Finger können sich quer, insbesondere senkrecht, zu der Längsrichtung der Fluidleitung erstrecken.

Bei einer ganz besonderen Ausführung kann mittels der Aufsatzvorrichtung wahlweise ein Durchmischen der flüssigen Probe oder ein Einsaugen der flüssigen Probe in die Fluidleitung oder ein Dispensieren von Fluid in die flüssige Probe durchgeführt werden. Somit können mit der Aufsatzvorrichtung unterschiedliche Prozessierungsschritte ausgeführt werden.

Nach einem Unterbrechen des Durchmischens der flüssigen Probe kann nach Ablauf einer vorgegebenen Zeitdauer ein Teil der flüssigen Probe in die Fluidleitung eingesaugt werden. Dies ist insbesondere dann von Vorteil, wenn abgewartet werden soll, dass sich Feststoffe in der flüssigen Probe, wie beispielsweise Biomasse oder Zellen absetzen und somit nur der Überstand eingesaugt werden soll.

Alternativ kann unmittelbar nach dem Unterbrechen des Durchmischens ein Teil der flüssigen Probe in die Fluidleitung eingesaugt werden. Dies ist dann vorteilhaft, wenn ein Aliquot der flüssigen Probe aufgenommen werden soll.

Nach einem Einsaugen eines Teils der flüssigen Probe kann die Fluidleitung aus der flüssigen Probe herausgezogen und die Aufsatzvorrichtung, insbesondere die Fluidleitung, von dem Behältnis wegtransportiert werden. Dies kann manuell durch den Benutzer oder automatisch durch eine Transportvorrichtung erfolgen. Die flüssige Probe ist in der Fluidleitung festgelegt und kann nicht von selbst aus der Fluidleitung ausströmen. So kann die Aufsatzvorrichtung zu einem Laborgerät transportiert werden, in das die in der Fluidleitung befindliche flüssige Probe dispensiert wird.

Alternativ kann die Fluidleitung zu einem anderen Behältnis transportiert werden. Die in der Fluidleitung befindliche flüssige Probe kann in das andere Behältnis dispensiert werden. Sofern in dem anderen Behältnis eine andere flüssige Probe enthalten ist, kann der in der Fluidleitung befindliche Teil der flüssigen Probe in die andere flüssige Probe dispensiert werden.

Bei Vorsehen eines Probenträgers der mehrere Behältnisses aufweist, kann die Aufsatzvorrichtung nach einem Herausziehen der Fluidleitung aus einem Behältnis des Probenträgers die Fluidleitung in eine andere Position bewegt werden, so dass die Fluidleitung in ein anderes Behältnis des Probenträgers eindringt und dort die in der Fluidleitung eingesaugte flüssige Probe dispensiert wird. Natürlich ist es auch möglich, dass bei einer Aufsatzvorrichtung, die mehrere Fluidleitungen aufweist, ein paralleles Einsaugen der flüssigen Proben oder des Gases und/oder ein paralleles Dispensieren der flüssigen Proben oder des Gases erfolgt. Im Ergebnis können mittels der Aufsatzvorrichtung gleichzeitig unterschiedliche Arbeitsschritte in unterschiedlichen Behältnissen durchgeführt werden.

Natürlich ist es alternativ möglich, dass in die Fluidleitung der Aufsatzvorrichtung zuerst eine Flüssigkeit aus einem externen, nicht zu der Vorrichtung oder der Aufsatzvorrichtung gehörenden Flüssigkeitstank eingesaugt wird. Die Aufsatzvorrichtung wird anschließend zu dem Behältnis transportiert und die Flüssigkeit, insbesondere zum erstmaligen Befüllen, in das Behältnis dispensiert.

Bei einer besonderen Ausführung kann die Aufsatzvorrichtung, insbesondere die Fluidleitung, mit einer Pumpe fluidisch verbunden sein. Darüber hinaus kann nach einem Verbinden der Pumpe mit der Aufsatzvorrichtung die Fluidleitung, insbesondere alle Fluidleitungen, mit der Pumpe fluidisch verbunden sein. Eine derartige Ausführung bietet den Vorteil, dass eine fluidische Verbindung zwischen der Pumpe und der Fluidleitung oder den Fluidleitungen auf einfache Weise, ohne das weitere Schritte unmittelbar nach dem Anschließen der Pumpe an die Aufsatzvorrichtung notwendig sind, realisiert werden kann.

Bei einer ganz besonderen Ausführung kann bei einem Untersuchen der flüssigen Probe eine, insbesondere vorgegebene, Anzahl von Detektionsmitteln, insbesondere Mikropartikel und/oder Sensorspots, in das Behältnis vorgesehen werden, wobei die Detektionsmittel dazu bestimmt, sind eine chemische Spezies der flüssigen Probe zu binden und ihre optische Eigenschaften, wie beispielsweise die Fluoreszenz, die Farbe und/oder den Kontrast, aufgrund der Bindung zu ändern. Anschließend kann die optische Eigenschaft des Detektionsmittels ermittelt werden.

Im Anschluss daran kann mittels der ermittelten optischen Eigenschaft des Detektionsmittels die Eigenschaft der flüssigen Probe als Ermittlungsergebnis bestimmt werden und/oder mittels der ermittelten optischen Eigenschaft des Detektionsmittels kann die Präsenz und/oder Quantität einer in der flüssigen Probe enthaltenen Spezies als Ermittlungsergebnis bestimmt werden.

Die Vorrichtung kann eine optische Erfassungseinrichtung aufweisen, mittels der Eigenschaften der Probe erfasst werden können. Darüber hinaus kann mittels der optischen Erfassungseinrichtung die Präsenz und/oder die Quantität der in der flüssigen Probe enthaltenen Spezies bestimmt werden. Die optische Erfassungseinrichtung ist mit der Steuervorrichtung der Vorrichtung datentechnisch verbunden. Die optische Erfassungseinrichtung kann eine optische Abbildungsvorrichtung, insbesondere eine Kamera, aufweisen, mittels der eine Abbildung der flüssigen Probe erzeugt werden kann. Dies ist möglich, weil das Behältnis teilweise transparent ist.

Die optische Erfassungseinrichtung kann an einem von dem Aufsatz abgewandten Ende des Behältnisses angeordnet sein. Bei mehreren Behältnissen können mehrere optische Erfassungseinrichtungen vorgesehen werden, wobei jede optische Erfassungseinrichtung einem einzigen Behältnis zugeordnet ist. Somit können Abbildungen von jeder flüssigen Probe erzeugt werden.

Die detektierten Spezies können chemische Spezies in der flüssigen Probe, wie beispielsweise gelöste Gase, Biomoleküle, etc. sein. Ein Sensorspot kann eine funktionalisierte Oberfläche im Behältnis sein. Der Sensorspot kann an einem vorgegebenen Abschnitt des Behältnisses angeordnet sein. Die Mikropartikel können in die flüssige Probe zugegeben werden und/oder magnetisch ausgeführt sein. Dies bietet den Vorteil, dass vermieden werden kann, dass die Mikropartikel bei einem Einsaugen der flüssigen Probe in die Fluidleitung ebenfalls eingesaugt werden. Mikropartikel bieten gegenüber Sensorspots den Vorteil, dass sie mehr Moleküle binden können, da sie durch die gesamte flüssige Probe bewegt werden können.

Die flüssige Probe kann unter Berücksichtigung des Ermittlungsergebnisses überwacht werden. Insbesondere kann die Kulturbedingung, wie beispielsweise der pH-Wert und/oder der Sauerstoffgehalt, überwacht werden. So kann abhängig vom Ermittlungsergebnis ein Warnsignal an den Benutzer ausgegeben werden und/oder es können weitere Prozessierungsschritte eingeleitet werden. Darüber hinaus kann unter Berücksichtigung des Ermittlungsergebnisses die Fluidzufuhr in die flüssige Probe oder eine Fluidabfuhr aus der flüssigen Probe geregelt werden. So kann beispielsweise anhand des Ermittlungsergebnisses bestimmt werden, dass das Behältnis zu wenig Flüssigkeit aufweist. Daher kann mittels der Aufsatzvorrichtung neue Flüssigkeit in das Behältnis eingebracht werden. Alternativ kann mittels des Ermittlungsergebnisses festgestellt werden, dass der Gasgehalt der flüssigen Probe zu gering ist, so dass mittels der Aufsatzvorrichtung Gas in die flüssige Probe zugeführt wird. Darüber hinaus kann das Ermittlungsergebnis genutzt werden, um die erfolgversprechendste Zellkultur auszuwählen. Dabei ist eine Zellkultur umso erfolgsversprechender je höher die Anzahl der produzierten Biomoleküle sind.

Bei einer besonderen Ausführung kann in der Fluidleitung ein Filter angeordnet sein, der flüssigkeitsundurchlässig und gasdurchlässig ist. Durch das Vorsehen des Filters wird vermieden, dass die flüssige Probe in den Aufsatz strömen kann.

Dabei kann die Fluidleitung einstückig oder wieder lösbar mit dem Aufsatz ausgeführt sein. Darüber hinaus kann die Fluidleitung fluidisch mit dem Aufsatz verbunden sein. Der Aufsatz kann das Behältnis abdecken und/oder sich auf dem Behältnis abstützen. Darüber hinaus kann der Aufsatz mit dem Behältnis wieder lösbar verbunden sein.

Darüber hinaus kann die Ausführung einen Deckel aufweisen, der, insbesondere unmittelbar, an dem Behältnis anordenbar und/oder befestigbar ist. Insbesondere kann der Deckel auf dem Behältnis aufliegen. Der Deckel kann einen Durchbruch aufweisen, durch den sich die Fluidleitung erstreckt.

Die Fluidleitung kann pipettenförmig ausgeführt sein. Alternativ kann die Fluidleitung in Richtung zum Behältnis, insbesondere zu einem Behältnisboden, einen konstanten Querschnitt aufweisen. Darüber hinaus kann die Fluidleitung in Richtung zum Behältnis, insbesondere zu einem Behältnisboden, einen sich, insbesondere kontinuierlich, verjüngenden Querschnitt aufweisen. Auch kann die Fluidleitung mit ihrer Außenfläche an einer Innenwand des Behältnisses anliegen. Die Form der Fluidleitung, insbesondere der Durchmesser der Fluidleitung, kann so gewählt werden, dass die Strömungsgeschwindigkeit und die Menge der angesaugten flüssigen Probe ausreichend hoch sind, damit ein Durchmischen der Probe realisiert werden kann. Die Fluidleitung kann darüber hinaus derart ausgeführt sein, dass eine Außenseite der Fluidleitung hydrophob ausgeführt ist. Dadurch kann ein Anhaften von Flüssigkeitsresten an der Fluidleitung auf einfache Weise verhindert werden.

Der Aufsatz oder der Deckel können das Behältnis dichtend abschließen. Insbesondere kann der Aufsatz oder der Deckel eine Dichtung, wie beispielsweise einen O-Ring, aufweisen. Somit kann verhindert werden, dass die flüssige Probe aus dem Behältnis verdunstet.

Bei einer ganz besonderen Ausführung kann die Aufsatzvorrichtung wenigstens ein Ventil aufweisen, mittels dem die Fluidleitung verschließbar ist. Somit kann mittels des Ventils gesteuert, ob das Fluid, insbesondere Gas, zu der Fluidleitung zugeführt wird. Das Ventil kann mit der Steuervorrichtung verbunden sein und die Ventilstellung kann durch die Steuervorrichtung gesteuert werden. Bei Vorsehen von mehreren Fluidleitungen kann jeder Fluidleitung ein Ventil zugeordnet sein. Die Ventile können jeweils mit der Steuervorrichtung verbunden sein, so dass die Steuervorrichtung die Ventilstellung des jeweiligen Ventils steuern kann.

Bei einer ganz besonderen Ausführung kann die Aufsatzvorrichtung wenigstens eine weitere Fluidleitung aufweisen, die in die flüssige Probe ragt und durch die ein weiteres Fluid in die Probe dispensiert wird. Dies bedeutet, dass die weitere Fluidleitung in dasselbe Behältnis eindringt wie die Fluidleitung. Das weitere Fluid kann identisch zu dem Fluid sein. Alternativ kann das dispensierte Fluid dem Teil der zuvor eingesaugten flüssigen Probe entsprechen und das weitere Fluid kann das Gas sein. Bei dieser Ausführung kann mittels der Fluidleitung ein Teil der flüssigen Probe eingesaugt oder der eingesaugte Teile dispensiert werden und mittels der weiteren Fluidleitung Gas in das Behältnis dispensiert werden.

Die Aufsatzvorrichtung kann wenigstens eine andere Fluidleitung aufweisen, die in eine andere flüssige Probe eines anderen Behältnisses ragt, wobei die Fluidleitung und die andere Fluidleitung fluidisch verbunden sind und in die Fluidleitung die flüssige Probe und in die andere Fluidleitung die andere flüssige Probe derart angesaugt wird, dass die flüssige Probe nicht mit der anderen flüssigen Probe vermischt wird. Dazu kann die Steuervorrichtung vorgesehen sein, die beispielsweise die Pumpe derart steuert, dass keine Vermischung der flüssigen Probe mit der anderen Probe erfolgt. Somit kann auf besonders einfache Weise eine ungewünschte Vermischung der flüssigen Probe mit der anderen flüssigen Probe vermieden werden. Die flüssige Probe und die andere flüssige Probe können identisch sein. Alternativ können sich die flüssige Probe und die andere flüssige Probe voneinander unterscheiden.

Von besonderem Vorteil ist eine Vorrichtung, bei der die Aufsatzvorrichtung mit dem Behältnis befestigt ist. Die Aufsatzvorrichtung, insbesondere die Fluidleitung oder die Fluidleitungen, kann mit der Pumpe fluidisch verbunden sein. Dabei kann die Pumpe derart ausgeführt sein, dass das Einsaugen des Fluids und das Dispensieren des Fluids durch reziprokes Bewegen eines Pumpenelements realisiert werden. Die Pumpe kann als pneumatische Pumpe oder eine peristaltische Pumpe oder eine Piezomikropumpe ausgeführt sein.

Die Vorrichtung kann auch mehrere Pumpen aufweisen. Dies ist insbesondere bei einer Aufsatzvorrichtung mit mehreren Fluidleitungen von Vorteil, bei der die Fluidleitungen nicht miteinander fluidisch verbunden sind. So können wenigstens eine Fluidleitung mit einer Pumpe und wenigstens eine andere Fluidleitung mit einer anderen Pumpe fluidisch verbunden sein.

Die Fluidleitung kann mittels eines Fluidkanals mit der Pumpe fluidisch verbunden sein. Sofern die Pumpe nicht unmittelbar mit dem Fluidkanal, sondern mittels eines Schlauchs mit der Pumpe fluidisch verbunden ist, kann der Fluidkanal mittels des Schlauchs mit der Pumpe verbunden sein. Die andere Fluidleitung kann mittels eines anderen Fluidkanals mit der anderen Pumpe fluidisch verbunden sein. Sofern die andere Pumpe nicht unmittelbar mit dem Fluidkanal, sondern mittels eines anderen Schlauchs mit der Pumpe fluidisch verbunden ist, kann der andere Fluidkanal mittels des anderen Schlauchs mit der anderen Pumpe verbunden sein. Dabei kann der Fluidkanal und/oder der andere Fluidkanal in dem Aufsatz angeordnet sein. Der Fluidkanal kann mit mehreren Fluidleitungen fluidisch verbunden sein und/oder der andere Fluidkanal kann mit mehreren anderen Fluidleitungen fluidisch verbunden sein. Im Ergebnis wird eine einfach aufgebaute Aufsatzvorrichtung bereitgestellt.

Bei einer ganz besonderen Ausführung kann ein Probenträger mehrere Behältnisse aufweisen. Das Behältnis kann ein Volumen von 100 µl aufweisen. Der Probenträger kann eine Mikrotiterplatte sein. Die Mikrotiterplatte kann eine Platte mit 6 oder 24 oder 96 oder 384 oder 3456 sein. Natürlich kann die Mikrotiterplatte auch mehr Behältnisse aufweisen. Dabei kann die Mikrotiterplatte eine rechteckige Platte sein und/oder aus Kunststoff bestehen. Die voneinander isolierten Behältnisse können in Reihen und Spalten angeordnet sein. In den einzelnen Behältnissen können unterschiedliche flüssige Proben enthalten sein.

Der Probenträger ist derart ausgeführt, dass bei einem Entfernen der Aufsatzvorrichtung die Behältnisse untereinander nicht fluidisch verbunden sind. Insbesondere sind keine Fluidleitungen in Wänden des Probenträgers vorhanden, über die wenigstens zwei Behältnisse fluidisch miteinander verbunden sind.

Die Aufsatzvorrichtung kann mehrere Fluidleitungen aufweisen, die sich von dem Aufsatz in die gleiche Richtung erstrecken. Insbesondere kann jede Fluidleitung identisch wie die oben beschriebene Fluidleitung ausgeführt sein. Darüber hinaus kann jede der Fluidleitungen in ein Behältnis des Probenträgers eindringen. Dabei können natürlich mehrere Fluidleitungen in dasselbe Behältnis eindringen.

Der Fluidkanal, insbesondere die Fluidkanäle, in dem Aufsatz kann derart ausgebildet und ausgeführt sein, dass in dem und/oder durch den Fluidkanal ausschließlich Gas strömt. Dies bedeutet, dass der Fluidkanal, insbesondere die Fluidkanäle, derart ausgeführt sind, dass durch den Fluidkanal keine Flüssigkeit strömt. Somit kann auf einfache Weise eine Flüssigkeitsströmung zwischen zwei Behältnissen vermieden werden. Insbesondere kann der Fluidkanal, insbesondere die Fluidkanäle, derart ausgebildet und ausgeführt sein, dass bei gleicher Pumpenleistung bei der Förderung von Gas oder Flüssigkeit nur das Gas durch den Fluidkanal, insbesondere die Fluidkanäle, strömen kann.

Dies kann dadurch erreicht werden, dass der Fluidkanal, insbesondere die Fluidkanäle, derart ausgebildet sind, dass sie einen so hohen fluidischen Widerstand aufweisen, dass ausschließlich Gas durch den Fluidkanal, insbesondere die Fluidkanäle, strömen kann. Ein hoher fluidischer Widerstand lässt sich beispielsweise durch eine geeignete Ausbildung des Fluidkanals, insbesondere der Fluidkanäle, erreichen. Darüber hinaus kann die Fluidoberfläche des Fluidkanals, insbesondere der Fluidkanäle, die mit dem Fluid in Kontakt gelangt, derart ausgebildet sein, dass sie einer Benetzung entgegenwirkt, was ebenfalls einen Widerstand für eine Flüssigkeitsströmung darstellt und diese letztendlich verhindert.

Die Aufsatzvorrichtung kann derart ausgeführt sein, dass sich die mittels der einzelnen Fluidleitungen durchgeführten Prozessierungsschritte voneinander unterscheiden. So kann mittels einer Fluidleitung der Aufsatzvorrichtung, die in ein Behältnis eindringt, ein Gas in die flüssige Probe dispensiert werden. Bei einer ersten anderen Fluidleitung, die in ein erstes anderes Behältnis eindringt, kann ein Teil der anderen flüssigen Probe in die andere Fluidleitung eingesaugt und/oder dispensiert werden. Außerdem kann mittels einer zweiten anderen Fluidleitung, die in ein zweites anderes Behältnis eindringt, ein Durchmischen der weiteren flüssigen Probe durch abwechselndes Einsaugen und Dispensieren, insbesondere von Gas oder Fluid, realisiert werden. Dies ist möglich, da die Aufsatzvorrichtung wenigstens ein, insbesondere mehrere, Ventil aufweisen kann, wobei die Steuervorrichtung die Ventilstellung steuern kann und/oder die einzelnen Fluidleitungen mit unterschiedlichen Fluidkanälen fluidisch verbunden sind. Die unterschiedlichen Fluidkanäle können mit unterschiedlichen Pumpen fluidisch verbunden sein. Natürlich ist es möglich, dass in das Behältnis, das erste andere Behältnis und/oder zweite andere Behältnis mehrere Fluidleitungen eindringen.

In den Figuren ist der Erfindungsgegenstand schematisch dargestellt, wobei gleiche oder gleichwirkende Bauteile zumeist mit den gleichen Bezugszeichen versehen sind. Dabei zeigt:
- Fig. 1: eine schematische Darstellung einer Vorrichtung mit einer Aufsatzvorrichtung gemäß einem ersten Ausführungsbeispiel und einem Behältnis,
- Fig. 2: eine schematische Darstellung der Vorrichtung mit der Aufsatzvorrichtung gemäß dem ersten Ausführungsbeispiel und einem Behältnis, wobei mittels einer Fluidleitung Gas zugeführt wird,
- Fig. 3: eine schematische Darstellung einer Vorrichtung mit einer Aufsatzvorrichtung gemäß einem zweiten Ausführungsbeispiel und einem Behältnis,
- Fig. 4: eine schematische Darstellung einer Vorrichtung mit einer Aufsatzvorrichtung gemäß einem dritten Ausführungsbeispiel und einem Behältnis,
- Fig. 5: eine schematische Darstellung einer Vorrichtung mit einer Aufsatzvorrichtung gemäß einem vierten Ausführungsbeispiel und einem Behältnis,
- Fig. 6: eine schematische Darstellung einer Vorrichtung mit einer Aufsatzvorrichtung gemäß einem fünften Ausführungsbeispiel und einem Behältnis,
- Fig. 7: eine schematische Darstellung einer Vorrichtung mit einer Aufsatzvorrichtung gemäß einem sechsten Ausführungsbeispiel und einem Behältnis,
- Fig. 8: eine schematische Darstellung einer Vorrichtung mit einer Aufsatzvorrichtung gemäß einem siebten Ausführungsbeispiel und einem Behältnis,
- Fig. 9: eine schematische Darstellung einer Vorrichtung mit einer Aufsatzvorrichtung gemäß einem achten Ausführungsbeispiel und einem Behältnis,
- Fig. 10: eine schematische Darstellung einer Vorrichtung mit einer Aufsatzvorrichtung gemäß einem neunten Ausführungsbeispiel und einem Behältnis,
- Fig. 11: eine Draufsicht auf die in Fig. 10 dargestellte Vorrichtung,
- Fig. 12: eine Explosionsdarstellung einer Vorrichtung mit einer Aufsatzvorrichtung gemäß einem zehnten Ausführungsbeispiel und einer Mikrotiterplatte,
- Fig. 13: eine perspektivische Darstellung der in Figur 12 gezeigten Vorrichtung mit der Aufsatzvorrichtung und der Mikrotiterplatte im zusammengebauten Zustand,
- Fig. 14: eine Seitenschnittansicht der in Figur 12 gezeigten Vorrichtung mit der Aufsatzvorrichtung und der Mikrotiterplatte,

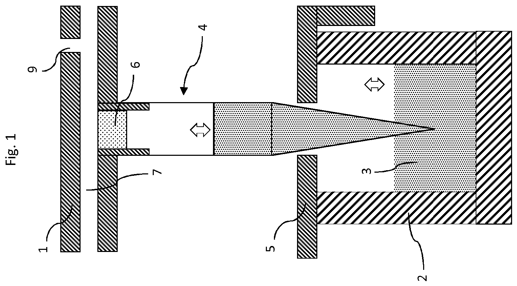

Die in Figur 1 gezeigte Vorrichtung weist eine Aufsatzvorrichtung gemäß einem ersten Ausführungsbeispiel und ein Behältnis 2 auf, wobei das Behältnis 2 eine flüssige Probe 3 aufnimmt. Die Aufsatzvorrichtung ist an dem Behältnis 2 wieder lösbar befestigt. Dabei weist die Aufsatzvorrichtung eine Fluidleitung 4 auf, die derart ausgebildet und dazu bestimmt ist, dass sie in die flüssige Probe 3 ragt.

Durch die Fluidleitung 4 kann ein Fluid, insbesondere ein zuvor eingesaugter Teil der flüssigen Probe, unmittelbar in die flüssige Probe 3 dispensiert werden und/oder ein Teil der flüssigen Probe 3 kann in die Fluidleitung 4 eingesaugt werden. Das Einsaugen und Dispensieren kann abwechselnd und/oder mehrmals hintereinander erfolgen. Somit kann der Stand der flüssigen Probe 3 innerhalb der Fluidleitung 4 und des Behältnisses 2 variieren, was in Figur 1 jeweils durch den Doppelpfeil symbolisiert ist. Infolge des Vorgangs des Einsaugens und Dispensierens wird eine Durchmischung der in dem Behältnis 2 befindlichen flüssigen Probe 3 erreicht.

Die Aufsatzvorrichtung weist einen Aufsatz 1, der mit der Fluidleitung 4 fluidisch verbunden ist und einen Deckel 5 auf, der das Behältnis 2 abdeckt und mit dem Behältnis 2 unmittelbar verbunden ist. Der Deckel 5 weist einen Durchbruch 8 auf, durch den sich die Fluidleitung 4 hindurch erstreckt, um in die flüssige Probe 3 einzutauchen. Die Fluidleitung 4 stützt sich, insbesondere in vertikaler Richtung, auf dem Deckel 5 ab, so dass der Aufsatz 1 mittelbar über die Fluidleitung 4 auf dem Behältnis 2 aufgesetzt ist. Die Fluidleitung 4 ist mit dem Aufsatz 1 wieder lösbar verbunden.

Innerhalb der Fluidleitung 4 ist ein Filter 6 angeordnet. Der Filter 6 ist flüssigkeitsundurchlässig und gasdurchlässig ausgeführt. Dies bedeutet, dass der in die Fluidleitung 4 eingesaugte Teil der flüssigen Probe 3 nicht durch den Filter 6 hindurch strömen kann. Jedoch kann ein Gas durch den Filter 6 hindurch strömen. Der Filter 6 ist in einem von der flüssigen Probe 3 entfernten Ende der Fluidleitung 4 angeordnet.

Der Aufsatz 1 weist einen Fluidkanal 7 auf, der mit der Fluidleitung 4, insbesondere mit einem in der Fluidleitung 4 befindlichen Kanal, fluidisch verbunden ist. Der Fluidkanal 7 ist außerdem mit einer Öffnung 9 im Aufsatz 1 fluidisch verbunden. Der Aufsatz 1 ist mittels der Öffnung 9 mit einer nicht dargestellten Pumpe fluidisch verbunden. Mittels der Pumpe kann der Druck in dem Fluidkanal 7 und damit der Fluidleitung 4 variiert werden, um ein Einsaugen eines Teils der flüssigen Probe 3 in die Fluidleitung 4 oder ein Dispensieren des eingesaugten Teils der flüssigen Probe 3 in das Behältnis zu bewirken.

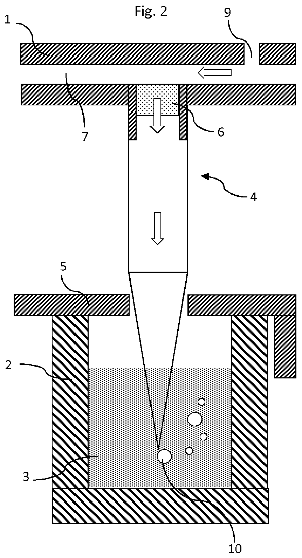

Die in Figur 2 dargestellte Aufsatzvorrichtung unterscheidet sich von der in Figur 1 beschriebenen Aufsatzvorrichtung lediglich in ihrer Betriebsweise. So wird bei der in Figur 2 dargestellten Aufsatzvorrichtung mittels der Fluidleitung 4 Gas in die flüssige Probe 3 zugeführt. Das Gas strömt entlang der Richtung der eingezeichneten Einfachpfeile über die Öffnung 9 in den Fluidkanal 7 und von dort in die Fluidleitung 4 und die flüssige Probe 3. Dabei strömt das Gas durch den Filter 6 hindurch. Bei dieser Betriebsweise erfolgt somit kein mehrmaliges und/oder abwechselndes Einsaugen und Dispensieren um die flüssige Probe 3 zu durchmischen. Ziel dieser Betriebsweise ist es nämlich, den Gasgehalt der flüssigen Probe 3 einzustellen.

Die nachstehend beschriebenen Ausführungsbeispiele können analog zu dem in den Figuren 1 und 2 dargestellten Ausführungsbeispiel mit den beiden zuvor beschriebenen Betriebsweisen betrieben werden.

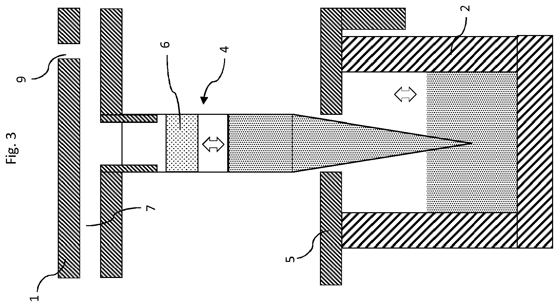

Figur 3 zeigt eine schematische Darstellung der Aufsatzvorrichtung gemäß einem zweiten Ausführungsbeispiel. Die Aufsatzvorrichtung unterscheidet sich von dem in Figur 1 und 2 dargestellten Ausführungsbeispiel in der Anordnung des Filters 6. So ist der Filter 6 in dem zweiten Ausführungsbeispiel nicht mehr am von der flüssigen Probe 3 entfernten Ende der Fluidleitung 4 angeordnet, sondern in einem Zwischenbereich der Fluidleitung 4.

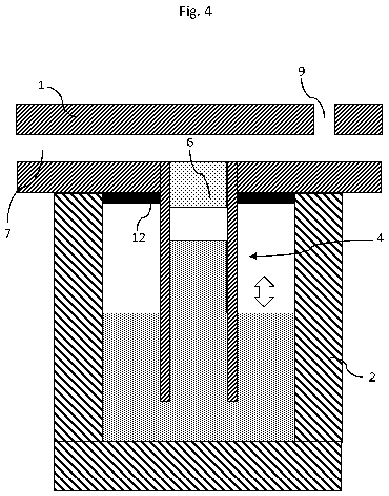

Figur 4 zeigt eine schematische Darstellung der Aufsatzvorrichtung gemäß einem dritten Ausführungsbeispiel. Die Aufsatzvorrichtung unterscheidet sich von dem in Figur 1 und 2 dargestellten Ausführungsbeispiel dadurch, dass die Aufsatzvorrichtung keinen Deckel 5 aufweist. So ist der Aufsatz 1 unmittelbar auf das Behältnis 2 gesetzt und mit diesem wieder lösbar verbunden. Darüber hinaus weist der Aufsatz 1 eine Dichtung 12 auf, mittels der das Behältnis 2 abgedichtet ist.

Ein weiterer Unterschied besteht in der Ausbildung der Fluidleitung 1. Während die in Figur 1 und 2 dargestellte Fluidleitung 4 pipettenförmig, mit einer sich zu der flüssigen Probe 3 verjüngenden Spitze, ausgebildet ist, weist die in Figur 4 dargestellte Fluidleitung 4 einen konstanten Querschnitt auf.

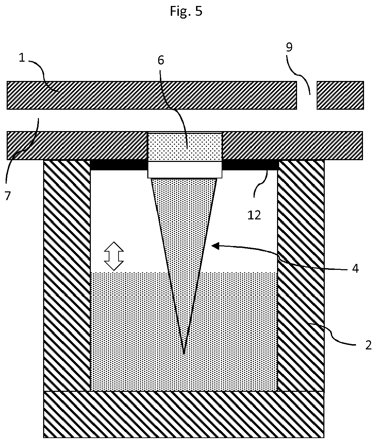

Figur 5 zeigt eine schematische Darstellung der Aufsatzvorrichtung gemäß einem vierten Ausführungsbeispiel. Dieses unterscheidet sich von dem in Figur 4 dargestellten Ausführungsbeispiel in der Ausbildung der Fluidleitung 4. So weist die Fluidleitung 4 in Richtung zur flüssigen Probe 3 einen sich kontinuierlich verjüngenden Querschnitt auf.

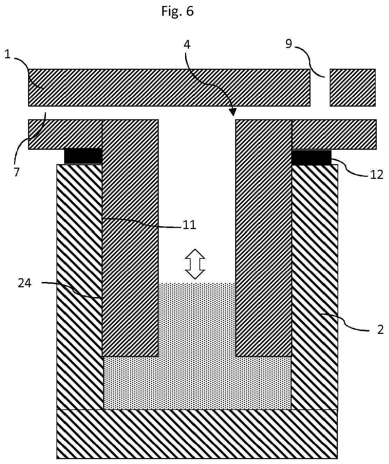

Figur 6 zeigt eine schematische Darstellung der Aufsatzvorrichtung gemäß einem fünften Ausführungsbeispiel. Dieses unterscheidet sich von dem in Figur 4 dargestellten dritten Ausführungsbeispiel in der Ausbildung der Fluidleitung 4. So ist die Fluidleitung 4 derart ausgeführt, dass ihre Außenseite 11, insbesondere eine Wandaußenseite der Fluidleitung 4, mit einer Innenseite 24 des Behältnisses 2 unmittelbar in Kontakt ist. Dabei weist die Fluidleitung 4 einen größeren Durchmesser auf, so dass in die Fluidleitung 4 eine größere Menge an flüssiger Probe 3 eingesaugt werden kann als in der in Figur 4 dargestellten Fluidleitung 4. Der Querschnitt der Fluidleitung 4 ist konstant.

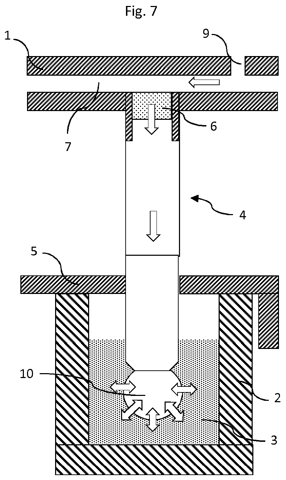

Figur 7 zeigt eine schematische Darstellung der Aufsatzvorrichtung gemäß einem sechsten Ausführungsbeispiel. Die Aufsatzvorrichtung unterscheidet sich von dem in Figur 2 dargestellten Ausführungsbeispiel in der Ausbildung der Fluidleitung 4 und der Art und Weise wie das Gas in die flüssige Probe 3 zugeführt wird.

Ein Unterschied besteht darin, dass die Fluidleitung 4 einen nahezu konstanten Querschnitt aufweist. Insbesondere weist die in Figur 7 dargestellte Fluidleitung 4 an ihrem Auslass einen größeren Durchmesser auf als die in Figur 2 dargestellte Fluidleitung 4. Darüber hinaus wird bei dem in Figur 7 dargestellten Ausführungsbeispiel am Auslas der Fluidleitung 4 eine Gasblase 10 erzeugt. Dazu wird Gas entlang der Richtung der eingezeichneten Einfachpfeile über die Öffnung 9, den Fluidkanal 7 und die Fluidleitung 4 zum Auslass der Fluidleitung 4 zugeführt. Dabei kann es zu einem diffusiven Austausch zwischen der Gasblase 10 und der flüssigen Probe 3 kommen wie durch die Doppelpfeile symbolisiert ist. Somit wird im Gegensatz zu der in Figur 2 dargestellten Ausführung das dispensierte Gas am Auslass der Fluidleitung 4 gehalten und verhindert, dass es in der flüssigen Probe 3 aufsteigt.

Bei einer alternativen Betriebsweise kann die Aufsatzvorrichtung derart betrieben werden, dass der Durchmesser der Gasblase 10 vergrößert oder verkleinert wird. Zum Verkleinern wird wenigstens ein Teil des Gases der Gasblase 10 in die Fluidleitung 4 eingesaugt. Bei dieser Betriebsweise lässt sich eine Durchmischung der flüssigen Probe 3 durch Änderung des Gasblasendurchmessers realisieren.

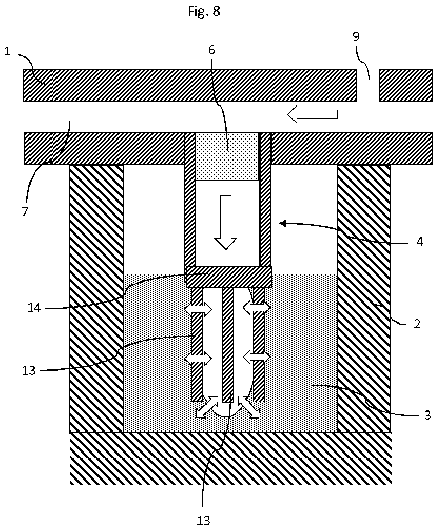

Die in Figur 8 dargestellte Aufsatzvorrichtung gemäß einem siebten Ausführungsbeispiel unterscheidet sich von der in Figur 4 dargestellten Ausführungsform darin, dass keine Dichtung vorhanden ist. Ein weiterer Unterschied besteht in der Ausbildung der Fluidleitung 4.

Die Fluidleitung 4 weist mehrere Finger 13 auf, die sich von einem Zwischenstück 14 der Fluidleitung 4 in Längsrichtung der Fluidleitung 4 erstrecken. Dabei sind die Finger 13 in Umfangsrichtung der Fluidleitung 4 benachbart und/oder beabstandet zueinander angeordnet. Dies bedeutet, dass in Umfangsrichtung gesehen jeweils ein Zwischenraum zwischen zwei Fingern 13 besteht. Die Finger 13 verhindern, dass das in die Fluidleitung 4 zugeführte Gas in der flüssigen Probe 3 aufsteigt. Somit kann ein diffusiver Austausch zwischen dem durch die Finger 13 festgehaltenen Gas und der flüssigen Probe 3, insbesondere über den Zwischenraum zwischen den Fingern 13 erfolgen, wie durch die Doppelpfeile symbolisiert ist. Das Gas wird ausgehend von der Öffnung 9 in Richtung der eingezeichneten Einfachpfeile zur flüssigen Probe 3 zugeführt.

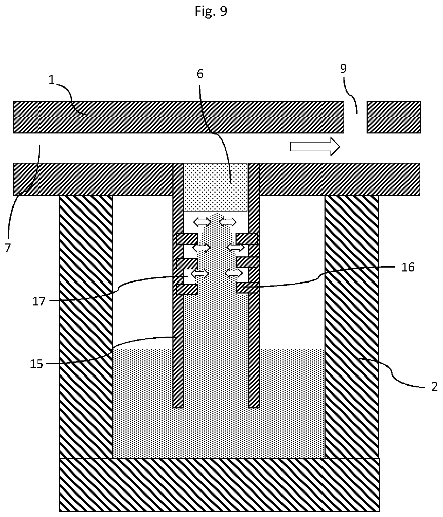

Die in Figur 9 dargestellte Aufsatzvorrichtung unterscheidet sich von der in Figur 8 dargestellten Aufsatzvorrichtung in der Ausbildung der Fluidleitung 4. So weist die Fluidleitung 4 keine Finger 13 auf, sondern mehrere senkrecht zur Längsachse der Fluidleitung 4 von einer Wand 15 der Fluidleitung 4 vorstehende, ringförmige Vorsprünge 16. Darüber hinaus sind die Vorsprünge 16 in Längsrichtung der Fluidleitung 4 benachbart und/oder beabstandet zueinander angeordnet.

Bei einem Ansaugen eines Teils der flüssigen Probe 3 dringt die flüssige Probe 3 in die Fluidleitung 4 ein. Dabei bildet sich zwischen zwei in Längsrichtung der Fluidleitung 4 benachbarten Vorsprüngen 16 jeweils ein Gasraum 17 in den die die flüssige Probe 3 nicht eindringt. Somit kann ein diffusiver Austausch zwischen der in die Fluidleitung 4 eingedrungen flüssigen Probe 3 und dem in dem Gasraum 17 befindlichen Gas erfolgen wie durch die Doppelpfeile symbolisiert ist. Zum Ansaugen des Teils der flüssigen Probe 3 wird das in der Fluidleitung 4 und/oder dem Fluidkanal 7 befindliche Gas in Richtung des Einfachpfeils über die Öffnung 9 abgesaugt.

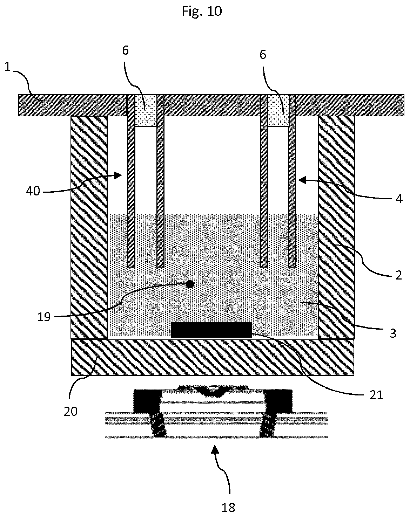

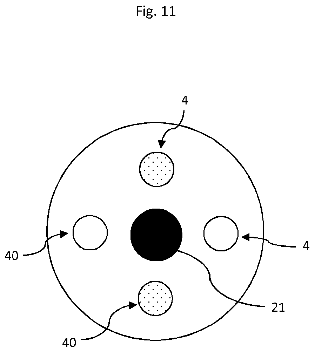

Figur 10 zeigt eine schematische Darstellung der Aufsatzvorrichtung gemäß einem neunten Ausführungsbeispiel. Figur 11 zeigt die Aufsatzvorrichtung von einer Draufsicht.

Die Aufsatzvorrichtung weist mehrere, insbesondere genau zwei Fluidleitungen 4 und mehrere, insbesondere genau zwei, weitere Fluidleitungen 40 auf. Sowohl die Fluidleitungen 4 als auch die weiteren Fluidleitungen 40 ragen in die flüssige Probe. Bei einer Betriebsweise der Aufsatzvorrichtung kann der flüssigen Probe durch die zwei weiteren Fluidleitungen 40 Gas zugeführt werden. Bei den restlichen zwei Fluidleitungen 4 kann jeweils ein abwechselndes Einsaugen eines Teils der flüssigen Probe und ein Dispensieren des zuvor eingesaugten Teils der flüssigen Probe erfolgen, um die flüssige Probe 3 zu durchmischen.
Auch wenn dies in den Figuren nicht dargestellt ist, sind die vier Fluidleitungen mit demselben im Aufsatz 1 befindlichen Fluidkanal 7 fluidisch verbunden. Insbesondere ist in Figur 10 nicht der vom Behältnis 2 abgewandte Teil des Aufsatzes 1 dargestellt, der den Fluidkanal 7 nach oben begrenzt. Natürlich sind auch andere Betriebsweisen möglich, bei denen der flüssigen Probe 3 Gas über weniger oder mehr als zwei weitere Fluidleitungen 40 zugeführt wird und/oder ein Durchmischen der flüssigen Probe 3 mittels Einsaugens und Dispensierens kann durch mehr oder weniger als zwei Fluidleitungen 4 realisiert werden.

Alternativ kann ein Durchmischen der flüssigen 3 Probe durch die zwei weiteren Fluidleitungen 40 realisiert werden, indem der Gasblasendurchmesser vergrößert und verkleinert wird. Gleichzeitig oder zeitlich versetzt kann mittels der Fluidleitungen 4 ein Durchmischen der flüssigen Probe 3 durch Einsaugen eines Teils der flüssigen Probe 3 und Dispensieren des eingesaugten Teils der flüssigen Probe 3 realisiert werden.

Die Vorrichtung weist neben der Aufsatzvorrichtung und dem Behältnis 2 auch eine optische Erfassungseinrichtung 18 zum Erfassen einer Eigenschaft der flüssigen Probe 3 auf. Die optische Erfassungseinrichtung 18 ist an einem von dem Aufsatz 1 abgewandten Ende des Behältnisses 2 angeordnet und kann eine optische Abbildungsvorrichtung, wie eine Kamera, aufweisen. Mittels der optischen Abbildungsvorrichtung kann eine Abbildung der flüssigen Probe 3 erzeugt werden.

Innerhalb der flüssigen Probe 3 sind Mikropartikel 19 angeordnet. Darüber hinaus befindet sich an einem Behältnisboden 20 ein Sensorspot 21. Die optische Erfassungseinrichtung 18 kann mittels der durch die optische Abbildungsvorrichtung erzeugten Abbildungen unter anderem das Vorliegen einer chemischen Spezies und/oder einige physikalische Eigenschaften der flüssigen Probe erfassen. Dieses Ergebnis kann an eine nicht dargestellte Steuervorrichtung übermittelt werden.

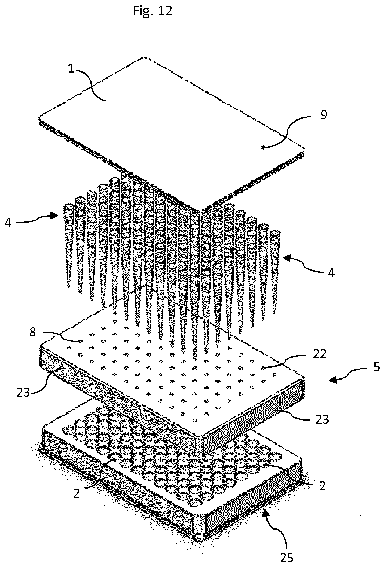

Figur 12 zeigt eine Explosionsdarstellung einer Vorrichtung mit einer Aufsatzvorrichtung gemäß einem zehnten Ausführungsbeispiel und einer Mikrotiterplatte 25. Die Aufsatzvorrichtung unterscheidet sich von den bisherigen Aufsatzvorrichtungen darin, dass sich von dem Aufsatz 1 eine Vielzahl von Fluidleitungen 4 in Richtung zur Mikrotiterplatte 25 erstrecken. Der Aufsatz 1 und/oder die Fluidleitungen 4 kann wie in einer in den Figuren 1 bis 11 offenbarten Ausführungsform ausgebildet sein. Darüber hinaus kann die in Figur 12 dargestellte Ausführungsform analog zu den in den Figuren 1 bis 11 beschriebenen Ausführungsformen betrieben werden.

Der Deckel 5 ist kastenförmig ausgebildet und weist eine Oberseite 22, die auf die Mikrotiterplatte 25 aufgesetzt wird, und Randabschnitte 23 auf, die sich von der Oberseite 22 in Richtung zur Mikrotiterplatte 25 erstrecken. Der Deckel 5 weist außerdem eine Vielzahl von Durchbrüchen 8 auf. Insbesondere entspricht die Anzahl der Durchbrüche 8 der Anzahl der Behältnisse 3 in der Mikrotiterplatte 25 und der Anzahl der Fluidleitungen 4. Die Mikrotiterplatte 25 weist eine Vielzahl von Behältnissen 2 auf, in der sich in der Figur nicht dargestellte flüssige Proben, wie beispielsweise Zellkulturen befinden. Die einzelnen Behältnisse 2 sind nicht miteinander fluidisch verbunden.

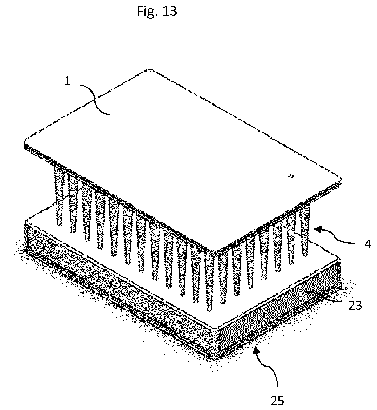

Wie aus Figur 13, die die Vorrichtung in einem zusammengebauten Zustand zeigt, ersichtlich ist, deckt der Deckel 5 alle Behältnisse 3 der Mikrotiterplatte 25 ab. Insbesondere ist der Deckel 5 derart ausgeführt, dass er unmittelbar auf die Mikrotiterplatte 25 gesetzt wird. Im Ergebnis kann durch den Deckel 5 verhindert werden, dass bei einem Durchmischen der in den Behältnissen 3 enthaltenen Proben diese aus den Behältnissen 3 herausströmen.

Jede der Fluidleitungen 4 erstreckt sich durch einen Durchgang 8 hindurch, um in das Behältnis 3 einzudringen. Der Aufsatz 1 ist oberhalb des Deckels 5 angeordnet und weist eine Öffnung 9 auf. Der Aufsatz 1 kann mittels der Öffnung 9 mit einer nicht dargestellten Pumpe fluidisch verbunden werden.

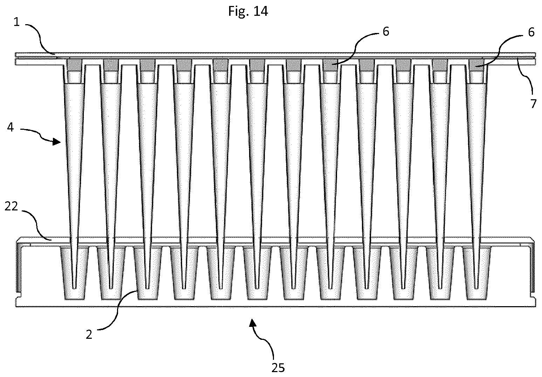

Wie aus Figur 14 ersichtlich ist, ist die Öffnung 9 mit dem in dem Aufsatz 1 befindlichen Fluidkanal 7 fluidisch verbunden. Der Fluidkanal 7 erstreckt sich durch den Aufsatz 1. Jede der Fluidleitungen 4 ist mit dem Fluidkanal 7 fluidisch verbunden. Natürlich sind auch Ausführungen denkbar, bei denen nicht alle Fluidleitungen mit dem Fluidkanal 7, sondern mit einem anderen nicht dargestellten Fluidkanal fluidisch verbunden sind. Dabei ist der andere Fluidkanal mit dem Fluidkanal 7 fluidisch nicht verbunden. In diesem Fall weist der Aufsatz 1 noch eine weitere nicht dargestellte Öffnung auf, die mit einer anderen nicht dargestellten Pumpe fluidisch verbunden ist. Die Aufsatzvorrichtung weist eine Vielzahl von in den Figuren nicht dargestellten Ventilen auf. Die Ventilstellung der einzelnen Ventile kann durch die nicht dargestellte Steuervorrichtung der Vorrichtung gesteuert werden. Mittels der Steuervorrichtung können die Ventile gezielt angesteuert werden, um eine Fluidströmung zu bestimmten Fluidleitungen 4 und damit zu bestimmten Behältnissen 2 zu realisieren.

Die Fluidleitungen 4 erstrecken sich jeweils unmittelbar vom Aufsatz 1 und sind mit diesem wieder lösbar verbunden. Dabei sind die Fluidleitungen 4 dazu bestimmt und entsprechend ausgebildet, dass sie jeweils in eine in dem Behältnis befindliche flüssige Probe 3 eintauchen. Die flüssige Probe ist in Figur 14 nicht dargestellt.

### Bezuqszeichenliste:

- 1: Aufsatz
- 2: Behältnis
- 3: flüssige Probe
- 4: Fluidleitung
- 5: Deckel
- 6: Filter
- 7: Fluidkanal
- 8: Durchbruch
- 9: Öffnung
- 10: Gasblase
- 11: Außenseite
- 12: Dichtung
- 13: Finger
- 14: Zwischenstück
- 15: Wand
- 16: Vorsprünge
- 17: Gasraum
- 18: optische Erfassungseinrichtung
- 19: Mikropartikel
- 20: Behältnisboden
- 21: Sensorspot
- 22: Oberseite
- 23: Randabschnitte
- 24: Innenseite
- 25: Mikrotiterplatte
- 40: weitere Fluidleitung

## Patentansprüche

1. Verfahren zum Prozessieren einer in einem Behältnis (2) befindlichen flüssige Probe (3), wobei an dem Behältnis (2) eine Aufsatzvorrichtung derart befestigt wird, dass wenigstens eine Fluidleitung (4) in die flüssige Probe (3) ragt und durch die Fluidleitung (4) ein Fluid unmittelbar in die flüssige Probe (3) dispensiert wird und/oder ein Teil der flüssigen Probe (3) in die Fluidleitung (4) eingesaugt wird, **dadurch gekennzeichnet, dass** das dispensierte Fluid ein zuvor eingesaugter Teil der flüssigen Probe (3) ist und/oder dass das dispensierte Fluid ein zuvor aus der flüssigen Probe (3) eingesaugtes Gas ist.

2. Verfahren nach Anspruch 1, dass
a. das Einsaugen und Dispensieren mehrmals hintereinander ausgeführt wird, um die flüssige Probe (3) zu durchmischen und/oder dass
b. das Einsaugen und Dispensieren abwechselnd ausgeführt wird, um die flüssige Probe (3) zu durchmischen.

3. Verfahren nach Anspruch 2, **dadurch gekennzeichnet, dass** die Menge der eingesaugten flüssigen Probe (3) zwischen 5% und 30% der Gesamtmenge der flüssigen Probe (3) beträgt und der Vorgang des Einsaugens und Dispensierens wenigstens 3 mal wiederholt wird.

4. Verfahren nach einem der Ansprüche 1 bis 3, **dadurch gekennzeichnet, dass**
a. eine Gasblase (10) erzeugt wird, wobei ein Gasblasendurchmesser vergrößert und verkleinert wird, um die flüssige Probe (3) zu durchmischen und/oder dass
b. ein Gasgehalt der flüssigen Probe (3) durch Zufuhr des Gases in die flüssige Probe (3) eingestellt wird und/oder dass
c. ein Gasgehalt der flüssigen Probe (3) durch diffusiven Austausch zwischen der flüssigen Probe (3) und dem Gas und/oder zwischen dem in der Fluidleitung befindlichen Gas und der flüssigen Probe (3) eingestellt wird und/oder dass
d. ein Gasgehalt der flüssigen Probe (3) durch diffusiven Austausch zwischen des in einem Abschnitt der Fluidleitung (4) befindlichen Gases und dem in die Fluidleitung (4) eingesaugten Teils der flüssigen Probe (3) eingestellt wird und/oder dass
e. wahlweise ein Durchmischen der flüssigen Probe (3) oder ein Einsaugen der flüssigen Probe (3) in die Fluidleitung (4) oder ein Dispensieren von Fluid aus der Fluidleitung (4) in die flüssige Probe (3) durchgeführt wird und/oder dass.
f. das Durchmischen der flüssigen Probe (3) unterbrochen wird und nach Ablauf einer vorgegebenen Zeitdauer der Teil der flüssigen Probe (3) in die Fluidleitung (4) eingesaugt wird oder dass das Durchmischen der flüssigen Probe (3) unterbrochen wird und unmittelbar nach dem Unterbrechen des Durchmischens der Teil der flüssigen Probe (3) in die Fluidleitung (4) eingesaugt wird.

5. Verfahren nach einem der Ansprüche 1 bis 4, **dadurch gekennzeichnet, dass**
a. nach einem Einsaugen des Teils der flüssigen Probe (3) die Fluidleitung (4) aus der flüssigen Probe (3) herausgezogen und von dem Behältnis (2) wegtransportiert wird , insbesondere und dass die Aufsatzvorrichtung, insbesondere die Fluidleitung (4), zu einem weiteren Behältnis (2) transportiert wird und die in der Fluidleitung (4) befindliche flüssige Probe (3) in das weitere Behältnis dispensiert wird, und/oder dass
b. die Aufsatzvorrichtung, insbesondere die Fluidleitung (4), mit einer Pumpe fluidisch verbunden wird, insbesondere und dass das Durchmischen der flüssigen Probe (3) durch reziprokes Pumpen realisiert wird, und/oder dass
c. nach einem Verbinden der Pumpe mit der Aufsatzvorrichtung die Fluidleitung (4), insbesondere alle Fluidleitungen der Aufsatzvorrichtung, mit der Pumpe fluidisch verbunden sind, insbesondere und dass das Durchmischen der flüssigen Probe (3) durch reziprokes Pumpen realisiert wird.

6. Verfahren nach einem der Ansprüche 1 bis 5, **dadurch gekennzeichnet, dass**
a. bei einem Untersuchen der flüssigen Probe (3) eine, insbesondere vorgegebene, Anzahl von Detektionsmitteln, insbesondere Mikropartikel (15) und/oder Sensorspots (21), in dem Behältnis (2), insbesondere der flüssigen Probe (3), vorgesehen werden, wobei die Detektionsmittel dazu bestimmt, sind eine chemische Spezies der flüssigen Probe (3) zu binden und ihre optische Eigenschaften aufgrund der Bindung zu ändern, und die optische Eigenschaft des Detektionsmittels ermittelt wird und mittels der ermittelten optischen Eigenschaft des Detektionsmittels eine Eigenschaft der flüssigen Probe (3) als Ermittlungsergebnis bestimmt wird und/oder mittels der ermittelten optischen Eigenschaft des Detektionsmittels die Präsenz und/oder Quantität einer in der flüssigen Probe (3) enthaltenen Spezies als Ermittlungsergebnis bestimmt wird, insbesondere und dass eine Zufuhr oder Abfuhr von Fluid in die flüssige Probe (3) unter Berücksichtigung des Ermittlungsergebnisses geregelt wird und/oder dass
b. die Aufsatzvorrichtung eine weitere Fluidleitung (40) aufweist, die in die flüssige Probe (3) ragt und durch die ein weiteres Fluid in die flüssige Probe (3) dispensiert wird und/oder dass
c. die Aufsatzvorrichtung eine andere Fluidleitung aufweist, die in eine andere flüssige Probe eines anderen Behältnisses ragt, wobei die Fluidleitung (4) und die andere Fluidleitung fluidisch verbunden sind, wobei in die Fluidleitung (4) ein Teil der flüssigen Probe (3) und in die andere Fluidleitung ein Teil der anderen flüssigen Probe derart angesaugt wird, dass die eingesaugte flüssige Probe (3) nicht mit der eingesaugten anderen flüssigen Probe vermischt wird.

7. Aufsatzvorrichtung, die an einem Behältnis (2) zur Aufnahme einer flüssigen Probe (3) wieder lösbar befestigbar ist, mit wenigstens einer Fluidleitung (4), die derart ausgebildet und dazu bestimmt ist, dass die Fluidleitung (4) in die flüssige Probe (3) ragt und dass durch die Fluidleitung (4) unmittelbar ein Fluid in die flüssige Probe (3) dispensierbar ist und/oder ein Teil der flüssigen Probe (3) in die Fluidleitung (4) einsaugbar ist, **dadurch gekennzeichnet, dass** die Aufsatzvorrichtung eine Steuervorrichtung aufweist, die ausgelegt ist, zu bewirken, dass das dispensierte Fluid ein zuvor eingesaugter Teil der flüssigen Probe ist und/oder dass das dispensierte Fluid ein zuvor aus der flüssigen Probe eingesaugtes Gas ist.

8. Aufsatzvorrichtung nach Anspruch 7, **dadurch gekennzeichnet, dass**
a. das dispensierte Fluid ein zuvor eingesaugtes Gas ist oder ein zuvor eingesaugter Teil der flüssigen Probe (3) ist und/oder dass
b. in der Fluidleitung (4) ein Filter (6) angeordnet ist, der flüssigkeitsundurchlässig und gasdurchlässig ist und/oder dass
c. die Aufsatzvorrichtung eine Steuervorrichtung aufweist, die ausgelegt ist, um das Einsaugen und Dispensieren mehrmals hintereinander und abwechselnd zu bewirken, um die flüssige Probe (3) zu durchmischen und/oder dass
d. die Aufsatzvorrichtung einen Aufsatz (1) aufweist, wobei die Fluidleitung (4) fluidisch mit dem Aufsatz (1) verbunden ist, insbesondere und dass der Aufsatz (1) oder der Deckel (5) eine Dichtung zum Abdichten des Behältnisses (2) aufweist, und/oder dass
e. die Aufsatzvorrichtung einen Aufsatz (1) aufweist, wobei die Fluidleitung (4) einstückig oder wieder lösbar mit dem Aufsatz (1) verbunden ist, insbesondere und dass der Aufsatz (1) oder der Deckel (5) eine Dichtung zum Abdichten des Behältnisses (2) aufweist, vorzugsweise und dass der Aufsatz (1) oder der Deckel (5) unmittelbar an dem Behältnis (2) anordenbar sind, und/oder dass
f. die Aufsatzvorrichtung einen Aufsatz (1) aufweist, wobei der Aufsatz (1) das Behältnis (2) abdeckt, insbesondere dass der Aufsatz (1) oder der Deckel (5) eine Dichtung zum Abdichten des Behältnisses (2) aufweist, vorzugsweise und dass der Aufsatz (1) oder der Deckel (5) unmittelbar an dem Behältnis (2) anordenbar sind, und/oder dass
g. die Aufsatzvorrichtung einen Aufsatz (1) aufweist, wobei der Aufsatz (1) wieder lösbar mit dem Behältnis (2) verbindbar ist, insbesondere und dass der Aufsatz (1) oder der Deckel (5) eine Dichtung zum Abdichten des Behältnisses (2) aufweist, vorzugsweise und dass der Aufsatz (1) oder der Deckel (5) unmittelbar an dem Behältnis (2) anordenbar sind.

9. Aufsatzvorrichtung nach Anspruch 7 oder 8, **dadurch gekennzeichnet, dass**
a. die Aufsatzvorrichtung einen Deckel (5) aufweist, der einen Durchbruch (8) aufweist, durch den sich die Fluidleitung (4) hindurch erstreckt und/oder dass
b. die Fluidleitung (4) pipettenförmig ausgeführt ist oder dass die Fluidleitung (4) in Richtung zum Behältnis (2) einen konstanten Querschnitt aufweist oder dass die Fluidleitung (4) in Richtung zum Behältnis (2) einen sich, insbesondere kontinuierlich, verjüngenden Querschnitt aufweist oder dass die Fluidleitung (4) mit ihrer Außenfläche an einer Innenwand des Behältnisses (2) anliegt und/oder dass
c. eine Wand der Fluidleitung (4) mehrere Vorsprünge (16) aufweist, die in Längsrichtung der Fluidleitung (4) voneinander beabstandet angeordnet sind und sich quer, insbesondere senkrecht, zu der Längsrichtung der Fluidleitung (4) erstrecken und/oder dass
d. sich von einer Wand der Fluidleitung (4) mehrere Finger (13) in Längsrichtung der Fluidleitung (4) erstrecken, wobei die Finger (13) in Umfangsrichtung der Fluidleitung (4) beabstandet zueinander angeordnet sind und/oder dass
e. eine Außenseite der Fluidleitung (4) hydrophob ausgebildet ist.

10. Aufsatzvorrichtung nach einem der Ansprüche 7 bis 9, **dadurch gekennzeichnet, dass**
a. die Aufsatzvorrichtung ein Ventil aufweist, mittels dem die Fluidleitung (4) verschließbar ist und/oder dass
b. die Aufsatzvorrichtung eine weitere Fluidleitung (40) aufweist, die in die flüssige Probe (3) ragt und durch die ein weiteres Fluid in das Behältnis (2) dispensierbar ist und/oder dass
c. die Aufsatzvorrichtung eine andere Fluidleitung aufweist, die in eine andere flüssige Probe eines anderen Behältnisses einbringbar ist, wobei die Fluidleitung (4) und die andere Fluidleitung fluidisch miteinander verbunden sind.

11. Vorrichtung mit einer Aufsatzvorrichtung nach einem der Ansprüche 7 bis 10 und einem Behältnis (2), **dadurch gekennzeichnet, dass** die Aufsatzvorrichtung an dem Behältnis (2) wieder lösbar befestigt ist.

12. Vorrichtung nach Anspruch 11, **dadurch gekennzeichnet, dass** die Aufsatzvorrichtung, insbesondere die Fluidleitung, mit einer Pumpe fluidisch verbunden ist, insbesondere und dass das Einsaugen des Fluids und das Dispensieren des Fluids durch reziprokes Bewegen eines Pumpenelements realisierbar ist und/oder dass die Pumpe eine pneumatische Pumpe oder eine peristaltische Pumpe oder eine Piezomikropumpe ist.

13. Vorrichtung nach Anspruch 12, **gekennzeichnet durch** eine mit der Pumpe verbundene Steuervorrichtung, die die Pumpe derart ansteuert, dass
a. das Einsaugen und Dispensieren mehrmals hintereinander ausgeführt wird, um die flüssige Probe (3) zu durchmischen und/oder dass
b. das Einsaugen und Dispensieren abwechselnd ausgeführt wird, um die flüssige Probe (3) zu durchmischen.

14. Vorrichtung nach einem der Ansprüche 11 bis 13, **dadurch gekennzeichnet, dass**
a. die Fluidleitung (4) mittels eines Fluidkanals (7) mit der Pumpe fluidisch verbunden ist, wobei der Fluidkanal (7) in dem Aufsatz (1) angeordnet ist und/oder dass
b. die Vorrichtung einen Gastank aufweist, der mit der Fluidleitung (4) fluidisch verbunden ist, insbesondere und dass die Vorrichtung eine Einstellvorrichtung aufweist, mittels der das in die Fluidleitung (4) zuführbare Gas einstellbar ist, und/oder dass
c. die Vorrichtung eine optische Erfassungsvorrichtung (18) zum Erfassen einer Eigenschaft der flüssigen Probe (3) aufweist, insbesondere und dass die optische Erfassungsvorrichtung (18) an einem von dem Aufsatz (1) abgewandten Ende des Behältnisses (2) angeordnet ist, und/oder dass
d. ein Probenträger, insbesondere eine Mikrotiterplatte, mehrere Behältnisse (2) aufweist und/oder dass
e. bei einer Demontage des Aufsatzes von dem Probenträger die Behältnisse (2) fluidisch miteinander nicht verbunden sind und/oder dass
f. die Steuervorrichtung die Pumpe derart ansteuert, dass in die Fluidleitung (4) ein Teil der flüssigen Probe (3) und in die andere Fluidleitung ein Teil der anderen flüssigen Probe derart angesaugt wird, dass die eingesaugte flüssige Probe (3) nicht mit der eingesaugten anderen flüssigen Probe vermischt wird und/oder dass
g. der Fluidkanal (4) in dem Aufsatz (1) derart ausgebildet und ausgeführt ist, dass durch den Fluidkanal (7) ausschließlich Gas strömt.

## Claims

1. A method for processing a liquid sample (3) in a container (2), an attachment device being secured to the container (2) in such a way that at least one fluid line (4) protrudes into the liquid sample (3) and a fluid is dispensed through the fluid line (4) directly into the liquid sample (3) and/or part of the liquid sample (3) is sucked into the fluid line (4), **characterised in that** the dispensed fluid is a previously sucked-in part of the liquid sample (3) and/or that the dispensed fluid is a gas previously sucked in from the liquid sample (3).

2. The method according to claim 1, in that
a. the sucking-in and dispensing is carried out multiple times in succession to mix the liquid sample (3) thoroughly and/or that
b. the sucking-in and dispensing is carried out alternately to mix the liquid sample (3) thoroughly.

3. The method according to claim 2, **characterised in that** the quantity of the liquid sample (3) sucked in is between 5% and 30% of the total quantity of the liquid sample (3) and the sucking-in and dispensing process is repeated at least 3 times.

4. The method according to any one of the claims 1 to 3, **characterised in that**
a. a gas bubble (10) is generated, wherein a gas bubble diameter is enlarged and reduced in size to mix the liquid sample (3) and/or that
b. a gas content of the liquid sample (3) is adjusted by feeding the gas into the liquid sample (3) and/or that
c. a gas content of the liquid sample (3) is adjusted by diffusive exchange between the liquid sample (3) and the gas and/or between the gas in the fluid line and the liquid sample (3) and/or that
d. a gas content of the liquid sample (3) is adjusted by diffusive exchange between the gas in a section of the fluid line (4) and the part of the liquid sample (3) sucked into the fluid line (4) and/or that
e. either the liquid sample (3) is mixed or the liquid sample (3) is sucked into the fluid line (4) or fluid is dispensed from the fluid line (4) into the liquid sample (3) and/or that.
f. the mixing of the liquid sample (3) is interrupted and after a predetermined period of time has elapsed the part of the liquid sample (3) is sucked into the fluid line (4) or that the mixing of the liquid sample (3) is interrupted and immediately after the interruption of the mixing the part of the liquid sample (3) is sucked into the fluid line (4).

5. The method according to any one of the claims 1 to 4, **characterised in that**
a. after the part of the liquid sample (3) has been sucked in, the fluid line (4) is withdrawn from the liquid sample (3) and transported away from the container (2), in particular and that the attachment device, in particular the fluid line (4), is transported to another container (2) and the liquid sample (3) in the fluid line (4) is dispensed into the other container, and/or that
b. the attachment device, in particular the fluid line (4), is fluidically connected to a pump, in particular and that the mixing of the liquid sample (3) is implemented by reciprocal pumping, and/or that
c. after connecting the pump to the attachment device, the fluid line (4), in particular all fluid lines of the attachment device, are fluidically connected to the pump, in particular and that the mixing of the liquid sample (3) is implemented by reciprocal pumping.

6. The method according to any one of the claims 1 to 5, **characterised in that**
a. when examining the liquid sample (3) an, in particular predetermined, number of detection means, in particular microparticles (15) and/or sensor spots (21), are provided in the container (2), in particular in the liquid sample (3), wherein the detection means are intended to bind a chemical species of the liquid sample (3) and change the optical properties thereof due to the binding, and the optical property of the detection means is determined and a property of the liquid sample (3) is determined as the determination result by means of the optical property of the detection means and/or the presence and/or quantity of a species contained in the liquid sample (3) is determined as the determination result by means of the determined optical property of the detection means, in particular and that a supply or discharge of fluid into the liquid sample (3) is regulated taking into account the determination result and/or that
b. the attachment device has a further fluid line (40) which protrudes into the liquid sample (3) and through which a further fluid is dispensed into the liquid sample (3) and/or that
c. the attachment device has another fluid line which protrudes into another liquid sample of another container, wherein the fluid line (4) and the other fluid line are fluidically connected, wherein a part of the liquid sample (3) is sucked into the fluid line (4) and a part of the other liquid sample is sucked into the other fluid line in such a manner that the sucked-in liquid sample (3) is not mixed with the other sucked-in liquid sample.

7. An attachment device which can be releasably secured to a container (2) for receiving a liquid sample (3), having at least one fluid line (4) which is designed and intended to ensure that the fluid line (4) protrudes into the liquid sample (3) and that a fluid can be dispensed into the liquid sample (3) directly through the fluid line (4) and/or a part of the liquid sample (3) can be sucked into the fluid line (4), **characterised in that** the attachment device has a control device which is designed to ensure that the dispensed fluid is a previously sucked-in part of the liquid sample and/or that the dispensed fluid is a gas that was previously sucked in from the liquid sample.

8. The attachment device according to claim 7, **characterised in that**
a. the dispensed fluid is a previously sucked-in gas or a previously sucked-in part of the liquid sample (3) and/or that
b. a filter (6) is arranged in the fluid line (4) which is impermeable to liquid and permeable to gas and/or that
c. the attachment device has a control device which is designed to effect the sucking-in and dispensing multiple times in succession and alternately, in order to mix the liquid sample (3) thoroughly and/or that
d. the attachment device has an attachment (1), wherein the fluid line (4) is fluidically connected to the attachment (1), in particular and that the attachment (1) or the lid (5) has a seal for sealing the container (2), and/or that
e. the attachment device has an attachment (1), wherein the fluid line (4) is integrally or releasably connected to the attachment (1), in particular and that the attachment (1) or the lid (5) has a seal for sealing the container (2), preferably and that the attachment (1) or the lid (5) can be arranged directly on the container (2), and/or that
f. the attachment device has an attachment (1), wherein the attachment (1) covers the container (2), in particular that the attachment (1) or the lid (5) has a seal for sealing the container (2), preferably and that the attachment (1) or the lid (5) can be arranged directly on the container (2), and/or that
g. the attachment device has an attachment (1), wherein the attachment (1) can be releasably connected to the container (2), in particular and that the attachment (1) or the lid (5) has a seal for sealing the container (2), preferably and that the attachment (1) or the lid (5) can be arranged directly on the container (2).

9. The attachment device according to claim 7 or 8, **characterised in that**
a. the attachment device has a lid (5) which has an opening (8) through which the fluid line (4) extends and/or that
b. the fluid line (4) is pipette-shaped or that the fluid line (4) has a constant cross-section in the direction of the container (2) or that the fluid line (4) has an, in particular continuously, tapering cross-section in the direction of the container (2) or that the fluid line (4) rests with the outer surface thereof on an inner wall of the container (2) and/or that
c. a wall of the fluid line (4) has a plurality of projections (16) which are arranged at a distance from one another in the longitudinal direction of the fluid line (4) and extend transversely, in particular perpendicularly, to the longitudinal direction of the fluid line (4) and/or that
d. a plurality of fingers (13) extend from a wall of the fluid line (4) in the longitudinal direction of the fluid line (4), wherein the fingers (13) are arranged at a distance from one another in the circumferential direction of the fluid line (4) and/or that
e. an outer side of the fluid line (4) is designed to be hydrophobic.

10. The attachment device according to any one of claims 7 to 9, **characterised in that**
a. the attachment device has a valve by means of which the fluid line (4) can be closed and/or that
b. the attachment device has an additional fluid line (40) which protrudes into the liquid sample (3) and through which an additional fluid can be dispensed into the container (2) and/or that
c. the attachment device has another fluid line which can be introduced into another liquid sample of another container, wherein the fluid line (4) and the other fluid line are fluidically connected to one another.

11. A device having an attachment device according to one of claims 7 to 10 and a container (2), **characterised in that** the attachment device is releasably secured to the container (2).

12. The device according to claim 11, **characterised in that** the attachment device, in particular the fluid line, is fluidically connected to a pump, in particular and that the sucking-in of the fluid and the dispensing of the fluid can be implemented by reciprocal movement of a pump element and/or that the pump is a pneumatic pump or a peristaltic pump or a piezo-micropump.

13. The device according to claim 12, **characterised by** a control device connected to the pump which controls the pump in such a manner that
a. the sucking-in and dispensing is carried out multiple times in succession to mix the liquid sample (3) and/or that
b. the sucking-in and dispensing is carried out alternately to mix the liquid sample (3) thoroughly.

14. The device according to one of claims 11 to 13, **characterised in that**
a. the fluid line (4) is fluidically connected to the pump by means of a fluid channel (7), the fluid channel (7) being arranged in the attachment (1) and/or that
b. the device has a gas tank which is fluidically connected to the fluid line (4), in particular and that the device has an adjusting device, by means of which the gas that can be fed into the fluid line (4) can be adjusted, and/or that
c. the device has an optical detection device (18) for detecting a property of the liquid sample (3), in particular and that the optical detection device (18) is arranged at an end of the container (2) facing away from the attachment (1), and/or that
d. a sample carrier, in particular a microtiter plate, comprises a plurality of containers (2) and/or that
e. when the attachment is detached from the sample carrier, the containers (2) are not fluidically connected to one another and/or that
f. the control device controls the pump in such a way that part of the liquid sample (3) is sucked into the fluid line (4) and part of the other liquid sample is sucked into the other fluid line, in such a manner that the sucked-in liquid sample (3) is not mixed with the other sucked-in liquid sample and/or that
g. the fluid channel (4) in the attachment (1) is designed and constructed in such a manner that only gas flows through the fluid channel (7).

## Revendications

1. Procédé de traitement d'un échantillon (3) liquide se trouvant dans un récipient (2), dans lequel un dispositif rapporté est fixé au récipient (2) de telle sorte qu'au moins une conduite de fluide (4) fait saillie dans l'échantillon (3) liquide et que, à l'aide de la conduite de fluide (4), un fluide est distribué directement dans l'échantillon (3) liquide et/ou une partie de l'échantillon (3) liquide est aspirée dans la conduite de fluide (4), **caractérisé en ce que** le fluide distribué est une partie préalablement aspirée de l'échantillon (3) liquide et/ou que le fluide distribué est un gaz préalablement aspiré de l'échantillon (3) liquide.

2. Procédé selon la revendication 1, **caractérisé en ce que**
a. l'aspiration et la distribution sont effectuées successivement plusieurs fois pour mélanger l'échantillon (3) liquide et/ou que
b. l'aspiration et la distribution s'effectuent alternativement pour mélanger l'échantillon (3) liquide.

3. Procédé selon la revendication 2, **caractérisé en ce que** la quantité d'échantillon (3) liquide aspiré est dans la plage comprise entre 5 % et 30 % rapporté à la quantité totale de l'échantillon (3) liquide et que le processus d'aspiration et de distribution est répété au moins 3 fois.

4. Procédé selon l'une quelconque des revendications 1 à 3, **caractérisé en ce que**
a. une bulle de gaz (10) est générée, dans lequel un diamètre de bulle de gaz est agrandi et réduit pour mélanger l'échantillon (3) liquide et/ou que
b. une teneur en gaz de l'échantillon (3) liquide par acheminement du gaz dans l'échantillon (3) liquide, est réglée et/ou que
c. une teneur en gaz de l'échantillon (3) liquide, par échange diffusif entre l'échantillon (3) liquide et le gaz et/ou entre le gaz dans la conduite de fluide et l'échantillon (3) liquide, est réglée et/ou que
d. une teneur en gaz de l'échantillon (3) liquide, par échange diffusif entre le gaz se trouvant dans une section de la conduite de fluide (4) et la partie de l'échantillon (3) liquide aspirée dans la conduite de fluide (4), est réglée et/ou que
e. soit un mélange de l'échantillon (3) liquide, soit une aspiration de l'échantillon (3) liquide dans la conduite de fluide (4), soit une distribution de fluide de la conduite de fluide (4) dans l'échantillon (3) liquide est effectuée et/ou que.
f. le mélange de l'échantillon (3) liquide est interrompu et après une durée prédéterminée, la partie de l'échantillon (3) liquide est aspirée dans la conduite de fluide (4) ou que le mélange de l'échantillon (3) liquide est interrompu et immédiatement après l'interruption du mélange, la partie de l'échantillon (3) liquide est aspirée dans la conduite de fluide (4).

5. Procédé selon l'une quelconque des revendications 1 à 4, **caractérisé en ce que**
a. après aspiration de la partie de l'échantillon (3) liquide, la conduite de fluide (4) est retirée de l'échantillon (3) liquide et transportée à l'écart du récipient (2), en particulier, et que le dispositif rapporté, en particulier la conduite de fluide (4), est transporté vers un autre récipient (2) et l'échantillon (3) liquide se trouvant dans la conduite de fluide (4) est distribué dans l'autre récipient, et/ou que
b. le dispositif rapporté, en particulier la conduite de fluide (4), est en liaison fluidique avec une pompe, en particulier, et que le mélange de l'échantillon (3) liquide est réalisé par pompage réciproque, et/ou que
c. après raccordement de la pompe au dispositif rapporté, la conduite de fluide (4), en particulier toutes les conduites de fluide du dispositif rapporté, sont raccordées de manière fluidique à la pompe, en particulier, et que le mélange de l'échantillon (3) liquide est réalisé par pompage réciproque.

6. Procédé selon l'une quelconque des revendications 1 à 5, **caractérisé en ce que**
a. lors de l'examen de l'échantillon (3) liquide, un nombre, en particulier prédéterminé, de moyens de détection, en particulier de microparticules (15) et/ou de points de capteur (21), sont situés dans le récipient (2), en particulier l'échantillon (3) liquide, dans lequel les moyens de détection sont conçus pour lier une espèce chimique de l'échantillon (3) liquide et pour modifier leurs propriétés optiques en raison de la liaison, et la propriété optique des moyens de détection est déterminée et, au moyen de la propriété optique déterminée des moyens de détection, une propriété de l'échantillon (3) liquide est définie en tant que résultat de la détermination et/ou au moyen de la propriété optique déterminée des moyens de détection, la présence et/ou la quantité d'une espèce contenue dans l'échantillon (3) liquide est définie en tant que résultat de la détermination, en particulier, et qu'un acheminement ou une décharge de fluide dans l'échantillon (3) liquide est régulée en tenant compte du résultat de la détermination et/ou que
b. le dispositif rapporté comporte une autre conduite de fluide (40), laquelle fait saillie dans l'échantillon (3) liquide et à travers laquelle un autre fluide est distribué dans l'échantillon (3) liquide et/ou que
c. le dispositif rapporté comporte une autre conduite de fluide, laquelle fait saillie dans un autre échantillon liquide d'un autre récipient, dans lequel la conduite de fluide (4) et l'autre conduite de fluide sont raccordées de manière fluidique, dans lequel, dans la conduite de fluide (4), une partie de l'échantillon (3) liquide, et, dans l'autre conduite de fluide, une partie de l'autre échantillon de liquide sont aspirées de telle sorte que l'échantillon (3) liquide aspiré n'est pas mélangé avec l'autre échantillon de liquide aspiré.

7. Dispositif rapporté, lequel peut être fixé de manière amovible, pour recevoir un échantillon (3) liquide, à un récipient (2), comprenant au moins une conduite de fluide (4), laquelle est conçue et destinée à ce que la conduite de fluide (4) fasse saillie dans l'échantillon (3) liquide et que, à l'aide de la conduite de fluide (4),un fluide peut être distribué directement dans l'échantillon (3) liquide et/ou une partie de l'échantillon (3) liquide peut être aspirée dans la conduite de fluide (4), **caractérisé en ce que** ledit dispositif rapporté comporte un dispositif de commande, lequel est conçu pour provoquer que le fluide distribué est une partie préalablement aspirée de l'échantillon liquide et/ou que le fluide distribué est un gaz qui préalablement aspiré à partir de l'échantillon liquide.

8. Dispositif rapporté selon la revendication 7, **caractérisé en ce que**
a. le fluide distribué est un gaz préalablement aspiré ou une partie préalablement aspirée de l'échantillon (3) liquide et/ou que
b. dans la conduite de fluide (4), un filtre (6) est disposé , lequel est imperméable aux liquides et perméable aux gaz et/ou que
c. le dispositif rapporté comporte un dispositif de commande, lequel est conçu pour effectuer l'aspiration et la distribution successivement plusieurs fois et alternativement afin de mélanger l'échantillon (3) liquide et/ou que
d. le dispositif rapporté comporte un élément rapporté (1), dans lequel la conduite de fluide (4) est en particulier raccordée de manière fluidique à l'élément rapporté (1) et que l'élément rapporté (1) ou le couvercle (5) comporte un joint d'étanchéité pour étanchéifier le récipient (2), et/ou que
e. le dispositif rapporté comporte un élément rapporté (1), dans lequel la conduite de fluide (4) est solidaire ou détachable de l'élément rapporté (1), en particulier, et que l'élément rapporté (1) ou le couvercle (5) comporte un joint d'étanchéité pour étanchéifier le récipient (2), de préférence, et que l'élément rapporté (1) ou le couvercle (5) peuvent être disposés directement sur le récipient (2), et/ou que
f. le dispositif rapporté comporte un élément rapporté (1), dans lequel l'élément rapporté (1) recouvre le récipient (2), en particulier que l'élément rapporté (1) ou le couvercle (5) comporte un joint d'étanchéité pour étanchéifier le récipient (2), de préférence et que l'élément rapporté (1) ou le couvercle (5) peuvent être disposés directement sur le récipient (2), et/ou que
g. le dispositif rapporté comporte un élément rapporté (1), dans lequel l'élément rapporté (1) peut être à nouveau raccordée de manière amovible au récipient (2), en particulier, et que l'élément rapporté (1) ou le couvercle (5) comporte un joint d'étanchéité pour étanchéifier le récipient (2), de préférence, et que l'élément rapporté (1) ou le couvercle (5) peuvent être disposés directement sur le récipient (2).

9. Dispositif rapporté selon la revendication 7 ou 8, **caractérisé en ce que**
a. le dispositif rapporté comporte un couvercle (5), lequel comporte une ouverture (8) à travers laquelle s'étend la conduite de fluide (4) et/ou que
b. la conduite de fluide (4) est conçue sous la forme de pipette ou que la conduite de fluide (4) comporte, dans la direction du récipient (2), une section transversale constante ou que la conduite de fluide (4) comporte une section transversale effilée, en particulier de manière continue, dans la direction du récipient (2) ou que la conduite de fluide (4) repose avec sa surface extérieure sur une paroi intérieure du récipient (2) et/ou que
c. une paroi de la conduite de fluide (4) comporte une pluralité de saillies (16), lesquelles sont disposées à distance les unes des autres dans la direction longitudinale de la conduite de fluide (4) et s'étendent transversalement, en particulier perpendiculairement, à la direction longitudinale de la conduite de fluide (4) et/ou que
d. plusieurs doigts (13) s'étendent depuis une paroi de la conduite de fluide (4) dans la direction longitudinale de la conduite de fluide (4), dans lequel les doigts (13) étant disposés à distance les uns des autres dans la direction circonférentielle de la conduite de fluide (4) et/ou que
e. un côté extérieur de la conduite de fluide (4) est conçue hydrophobe.

10. Dispositif rapporté selon l'une quelconque des revendications 7 à 9, **caractérisé en ce que**
a. le dispositif rapporté comporte une vanne au moyen de laquelle la conduite de fluide (4) peut être fermée et/ou que
b. le dispositif rapporté comporte une autre conduite de fluide (40), laquelle fait saillie dans l'échantillon (3) liquide et à travers laquelle un autre fluide est distribué dans le récipient (2) et/ou que
c. le dispositif rapporté comporte une autre conduite de fluide, laquelle peut être introduite dans un autre échantillon liquide d'un autre récipient, dans lequel la conduite de fluide (4) et l'autre conduite de fluide sont raccordées de manière fluidique l'une à l'autre.

11. Dispositif comprenant un dispositif rapporté selon l'une quelconque des revendications 7 à 10, et un récipient (2), **caractérisé en ce que** ledit dispositif rapporté est fixé de manière amovible au récipient (2).

12. Dispositif selon la revendication 11, **caractérisé en ce que** le dispositif rapporté, en particulier la conduite de fluide, est raccordé de manière fluidique à une pompe, en particulier, et que l'aspiration du fluide et la distribution du fluide peuvent être réalisées par mouvement réciproque d'un élément de pompe et/ou que la pompe est une pompe pneumatique ou une pompe péristaltique ou une micropompe piézo.

13. Dispositif selon la revendication 12, **caractérisé par** un dispositif de commande raccordé à la pompe, lequel commande la pompe de telle sorte que
a. l'aspiration et la distribution sont effectuées successivement plusieurs fois pour mélanger l'échantillon (3) liquide et/ou que
b. l'aspiration et la distribution s'effectuent alternativement pour mélanger l'échantillon (3) liquide.

14. Dispositif selon l'une quelconque des revendications 11 à 13, **caractérisé en ce que**
a. la conduite de fluide (4) est raccordée de manière fluidique à la pompe au moyen d'un canal de fluide (7), dans lequel le canal de fluide (7) est disposé dans l'élément rapporté (1) et/ou que
b. ledit dispositif comporte un réservoir de gaz, lequel est raccordé fluidiquement à la conduite de fluide (4), en particulier , et que ledit dispositif comporte un dispositif de réglage au moyen duquel le gaz pouvant être acheminé dans la conduite de fluide (4), peut être réglé, et/ou que
c. ledit dispositif comporte un dispositif de détection optique (18) pour détecter une propriété de l'échantillon (3) liquide, en particulier, et que le dispositif de détection optique (18) est disposé à une extrémité du récipient (2) opposée à l'élément rapporté (1), et/ou que
d. un porte-échantillon, en particulier une plaque de microtitrage, comporte plusieurs récipients (2) et/ou que
e. lors du démontage de l'élément rapporté du porte-échantillon, les récipients (2) ne sont pas raccordés de manière fluidique les uns aux autres et/ou que
f. le dispositif de commande commande la pompe de telle sorte que, dans la conduite de fluide (4),une partie de l'échantillon (3) liquide est aspirée et, dans l'autre conduite de fluide, une partie de l'autre échantillon liquide est aspirée de telle sorte que l'échantillon (3) liquide aspiré ne se mélange pas avec l'autre l'échantillon liquide et/ou que
g. le canal de fluide (4) dans l'élément rapporté (1) est conçu et construit de telle sorte que seul le gaz s'écoule à travers le canal de fluide (7).
